(19) Europäisches Patentamt / European Patent Office / Office européen des brevets

(11) **EP 3 168 211 A1**

(12) **EUROPEAN PATENT APPLICATION**


(43) Date of publication: **17.05.2017 Bulletin 2017/20**

(21) Application number: **15193955.0**

(22) Date of filing: **10.11.2015**

(51) Int Cl.:
*C07D 213/81* (2006.01)
*C07D 277/40* (2006.01)
*A61P 35/00* (2006.01)
*A61P 29/00* (2006.01)
*C07C 235/64* (2006.01)
*A61K 31/167* (2006.01)
*A61P 25/28* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**



(71) Applicant: **Westfälische Wilhelms-Universität Münster**
**48149 Münster (DE)**

(72) Inventors:
- **LÜDEKER, David**
  **58053 Hagen (DE)**
- **BRUNKLAUS, Gunther**
  **48161 Münster (DE)**

(74) Representative: **Kutzenberger Wolff & Partner**
**Theodor-Heuss-Ring 23**
**50668 Köln (DE)**


(54) **PHARMACEUTICAL CO-CRYSTALS OF NICLOSAMIDE**


(57) The invention relates to co-crystals of Niclosamide with co-formers selected from the group consisting of 2-aminothiazole, acetamide, benzamide, and isoniacide, pharmaceutical compositions and pharmaceutical dosage forms containing the co-crystals and medical uses thereof.


EP 3 168 211 A1

## Description

The invention relates to co-crystals of Niclosamide with co-formers selected from the group consisting of 2-aminothiazole, acetamide, benzamide, and isoniacide, pharmaceutical compositions and pharmaceutical dosage forms containing the co-crystals and medical uses thereof.

Niclosamide (2',5-dichloro-4'-nitrosalicylanilide, NCL) is a hydrophobic BCS class II taeniacide and restricted-used pesticide that may be applied to cure parasite infestations (including most tapeworms and cestoda) in both humans and animals or for water treatment against e.g. sea lampreys (*Petromyzon marinus*) or apple snails (*Pomacea canaliculcua*).

The primary effect of niclosamide comprises the inhibition of energy production in mitochondria, though niclosamide may also bind to DNA after its reductive activation rendering it possibly toxic to some aquatic organisms or plants. An early long term toxicology survey of molluscicides indicated that niclosamide neither has increased mutagenic, oncogenic or embryotoxic activity nor impact on liver and kidney functions. Despite that a single oral dose of niclosamide for adults in cestocidal treatment amounts to 2 g, niclosamide exhibits a very low acute toxicity in humans reflecting both its insufficient oral bioavailability (merely 10% in male Sprague-Dawley rats) and the fact that niclosamide is poorly absorbed from the intestinal tract. Recently identified promising properties of niclosamide include anti-inflammatory effects useful for the treatment of rheumatoid arthritis, prevention of protein aggregation in neurodegenerative diseases, or even for multi-targeted therapy of cancer and cancer stem cells (including an application of niclosamide as effective radio-sensitizer) (see e.g. Y. Li et al., Cancer Letters 349 (2014) 8-14).

However, the efficacy of niclosamide in medical treatments is currently limited by its insufficient solubility or bioavailability. Thus, research efforts are currently devoted to considerably improve the solubility (and thus bioavailability) of niclosamide thereby enhancing the achievable maximal serum concentrations and its efficacy in medical treatments.

In addition to suitable drug carriers such as polymeric particles or even supramolecular hosts introduced during formulation, either efficient crystallization screening (including polymorphs, salts, solvates or co-crystals or tailored amorphization of the active drug or its multi-component system of interest (co-amorphous drug systems) constitute two major approaches that are available for the manipulation of physicochemical properties of drugs. Though amorphous drugs may have great potential to reduce issues related to either poor dissolution rate or solubility-limited absorption in particular in case of drugs where salt formation cannot be applied, this approach is not readily accessible for the non-glass former niclosamide. Therefore, the various attempts to counteract niclosamides hydrophobic nature in order to facilitate better aqueous dispersion or solubility of niclosamide for example include salt formation of niclosamide with either ethanolamine or sodium (as part of a polymer-based controlled-release formulation) and inclusion of niclosamide in 4-sulphonatao-calix[n]arene or cyclodextrin host systems.

Since the niclosamide delivery formulations were mainly considered in the framework of water treatment such as control of snails in farm fields neither compatibility nor toxicity of certain additives with respect to the human metabolism was explicitly evaluated which only recently has changed due to the emerging interest in niclosamide as a potential candidate in cancer therapy. Consequently, chemically modified but highly water soluble derivatives of niclosamide, (e.g. with an attached phosphate group or alkylamino tethered derivatives) have been introduced, though at the expense of rather tedious synthetic protocols.

Moreover, the concept of hydrogen-bond mediated formation of pharmaceutical cocrystals was successfully applied to obtain co-compounds of niclosamide with suitable molecules taken from either the "Generally Regarded as Safe" (GRAS) or US FDA "Everything Added to Food in the United States" (EAFUS) list including caffeine (CAF), urea (URE), p-amino-benzoic acid (PABA), theophylline (THPH), nicotinamide (NA) or isonicotinamide (IA), respectively.

The co-crystals were designed according to the availability of specific supramolecular synthons and are mainly based on the robust {OH···O} synthon though in case of para-amino-benzoic acid the {OH···$NH_2$} synthon was identified. Since nicotinamide and its stereoisomer isonicotinamide contain $CONH_2$ and $N_{aromatic}$ functional groups, the likely resulting co-crystals may exhibit {OH···O}, {OH···$NH_2$}, or {OH···$N_{aromatic}$} synthons where the latter is a particularly versatile supramolecular unit for crystal engineering applications. In addition, salt co-crystals with concomitant presence of niclosamide both as neutral component and as salt co-former (derived from salification of niclosamide with inorganic salts) as well as mixed solvate/hydrate salts of niclosamide were very recently reported. The latter partially included the solvent dimethyl sulfoxide (DMSO), which in view of the identified brain degeneration due to apoptosis in the central nervous system induced by DMSO already at low doses of 0.3 mL/kg appears pharmaceutically questionable.

Rather well defined crystalline drugs are often preferred for the tailored design of solid oral dosage forms based on crystal engineering strategies. Niclosamide, however, is prone to solvate, hydrate or mixed solvate/hydrate formation even when reacted under solvent assisted solid grinding where only a few drops of suitable solvent are present. Thus, several solvates of niclosamide with methanol (MeOH), dimethyl sulfoxide (DMSO), N,N'-dimethyl formamide (DMF), diethyl ether ($Et_2O$) tetrahydrofuran (THF) and tetraethylene glycol (TEG), and the polymorphic monohydrates of niclosamide (NCL), i.e. NCL-$H_A$ and NCL-$H_B$ have been reported (though only single-crystal structures of niclosamide monohydrate $H_B$ and the solvates NCL-MeOH, NCL-THF or NCL-TEG are known to date), thereby clearly emphasizing the

necessity to utilize rather purified water-free solvents for the co-crystal synthesis of niclosamide to circumvent any hydrate by-products.

A less-defined hydration or dehydration of pharmaceutical solids ideally can be avoided during formulation development under controlled conditions but this may be more difficult upon prolonged storage of the final product, which then may adversely affect its physical, chemical or bio-medical properties. Similarly, charge-assisted synthons formed due to salt formation tend to be hygroscopic and should be avoided as well rendering co-crystallization attempts based on neutral synthons such as {OH···O} or {NH···O} rather preferred. Indeed, the molecular structure of niclosamide exhibits a planar, secondary amide group comprised of a C=O hydrogen bond acceptor and NH hydrogen bond donor. The latter forms an intramolecular S(6) hydrogen bonded ring with the oxygen atom of the neighboring hydroxyl group resulting in a rather exposed O-H unit that may be considered as suitable hydrogen donor for the successful co-crystal and/or solvate formation of niclosamide, as documented in the pristine compound niclosamide where this hydroxyl group connects via intermolecular $C_1^1(6)$ chains with the carbonyl hydrogen bond acceptor of an adjacent niclosamide moiety (note that niclosamide crystallizes in the monoclinic space group $P2_1/c$ with one molecule in the asymmetric unit).

DE 10 2007 030 695 relates to co-crystals of urea with amide- and/or urea-derivatives.

WO 2005/055983 pertains to a method of preparing mixed phase co-crystals of active agents with one or more materials that allows the modification of the active agent to a physical/crystal form useful for the delivery of the active agent, as well as compositions comprising the mixed phase co-crystals.

WO 2013/040187 discloses a stable ternary solid dispersion composition with enhanced physical stability and/or bioavailability comprising: (a) about 1% wt. to about 50% wt. of one or more poorly soluble active pharmaceutical ingredient (API) which belongs to Biopharmaceutics Classification System (BCS) class II and/or IV; (b) about 11% wt. to about 50% wt. of at least one water- soluble polymer; and (c) about 20% wt. to about 99% wt. of crosslinked polyvinylpyrrolidone (crospovidone, a water-insoluble polymer).

M.-Y. Bai et al., Colloids and Surfaces A: Physicochem. Eng. Aspects 419 (2013) 248-256 relates to the fabrication of niclosamide-suspension using an electrospray system to improve its therapeutic effects in ovarian cancer cells *in vitro.*

P. Sanphui et al., Crystal Growth & Design (2012), 12, 4588-4599 and F. Grifasi et al., Crystal Growth & Design (2015), 15, 1939-1948 disclose pharmaceutical co-crystals of niclosamide.

The niclosamide formulations according to the prior art are not satisfactory in every respect and there is a demand for formulations of niclosamide that are useful for medical treatments and that have an improved solubility.

It is an object of the invention to provide formulations of niclosamide that have advantages compared to the formulations of the prior art.

This object has been achieved by the subject-matter of the patent claims.

It has been found that co-crystals of niclosamide with certain co-formers have improved solubility. The inventors of the present invention have explored the potential of hydrogen-bond mediated co-crystal formation of niclosamide with suitable co-formers selected from the GRAS or EAFUS list, respectively. Indeed, solvent-assisted solid grinding and/or slow solvent evaporation yielded four new co-crystals:

(i) niclosamide - 2-aminothiazole (NCL-AT)

(ii) niclosamide - benzamide (NCL-BA),

(iii) niclosamide - isoniazide (NCL-IN), and

(iv) the polymorphic niclosamide - acetamide I and II (NCL-AA-I/NCL-AA-II).

Figures 1 to 11 show the following:

Figure 1: The molecular structures of niclosamide and the considered co-formers discussed in this study. All obtained co-crystals of niclosamide and the monohydrate have a (1:1) stoichiometry. Note that 2-aminothiazole derivatives were suggested as therapeutic leads for the treatment of prion diseases.

Figure 2: $^1$H MAS NMR spectra (acquired at 500.1 MHz and 30 kHz spinning frequency) of (a) niclosamide anhydrate (NCL) and the resulting (1:1) co-crystals (b) NCL-HA, (c) NCL-AA-I, (d) NCL-AA-II, (e) NCL-BA, (f) NCL-IN, (g) NCL-NA, (h) NCL-IA, and (i) NCL-AT respectively. The asterisks mark minor impurities due to residual solvents (either ethanol or ethylacetate).

Figure 3: Powder XRD pattern of (a) niclosamide anhydrate (NCL) in comparison to the novel co-crystals of NCL

with (b) benzamide (BA). (c) isoniazide (IN), respectively, and the reproduced co-crystals of NCL with (d) nicotinamide (NA) and (e) isonicotinamide (IA), since no explicit structure solution is provided.

Figure 4: The final Rietveld fit obtained for NCL-$H_A$: experimental data points, Rietveld refinement fit, background, difference $I_{obs}$- $I_{calc}$ and phase tick marks. The vertical line marks the place at which the data points are multiplied with factor five for better visibility of the data in the higher 20 region.

Figure 5: (left) NCL-$H_A$ hydrate forms trimeric layers involving the water molecule containing $C_2^2(8)$ and $C_2^2(14)$ chains. (right) π-π interactions (3.812 Å) lead to AAA packing of layers along the c-axis.

Figure 6: The final Rietveld fit obtained for NCL-AT; experimental data points, Rietveld refinement fit, background, difference $I_{obs}$ - $I_{calc}$ and phase tick marks. The vertical line marks the place at which the data points are multiplied with factor five for better visibility of the data in the higher 2 Θ region.

Figure 7: (left) The NCL-AT (1:1) co-crystal comprises a $C_2^2(10)$ chain motif formed by the hydroxyl group of NCL and the aromatic nitrogen of 2-aminothiazole as well as both the amino group of 2-aminothiazole and carbonyl oxygen of NCL, respectively; (right) π-π interactions (at a distance 3.645 Å) lead to ABAB packing of layers along the c-axis forming a γ-motif.

Figure 8: The final Rietveld fit of the co-crystal polymorph NCL-AA-I: experimental data points, Rietveld refinement fit, background, difference $I_{obs}$ - $I_{calc}$, phrase tick marks of NCL-AA-I and phase tick marks of NCL-AA-II. The vertical line marks the place at which the data points are multiplied with factor ten for better visibility of the data in the higher 20 region.

Figure 9: Final Rietveld fit of the co-crystal polymorph NCL-AA-II: experimental data points, Rietveld refinement fit, background, difference $I_{obs}$- $I_{calc}$, phase tick marks of NCL-AA-II and phase tick marks of NCL-AA-I. The vertical blue line marks the place at which the data points are multiplied with factor five for better visibility of the data in die higher 20 region.

Figure 10: (left top) The NCL-AA-I (1:1) co-crystal comprises a $R_4^4(20)$ ring motif between carbonyl oxygen of NCL and the amide group of acetamide connect ABAB layers of niclosamide. (right top). Within each layer of niclosamide molecules $C_2^2(12)$ chains link acetamide and NCL the nitro group and carbonyl oxygen of NCL and the amide group of acetamide. (bottom) Two antiparallel acetamide ribbons form a column in which NCL molecules are stacked through π-π-interactions at a distance of 3.404 Å.

Figure 11: (left top) The polymorph NCL-AA-II forms $D_2^3(7)$ hydrogen bonding motives between the hydroxyl group of NCL and the oxygen of acetamide containing acetamide $C_1^1(4)$ (right top). $C_1^1(4)$ columns of acetamide along the c-axis. (middle). Diagonal packing of NCL layers that are linked via π-π stacking (3.882 Å) in AAA fashion (bottom). Unit cell of the crystal structure viewed in the ab plane.

Figure 12 shows the numeration of atoms of NCL-$H_A$ for Table 5.

Figure 13 shows the numeration of atoms of NCL-AT for Table 6.

Figure 14 shows the numeration of atoms of NCL-AA-I for Table 7.

Figure 15 shows the numeration of atoms of NCL-AA-II for Table 8.

A first aspect of the invention relates a co-crystal or a multitude of co-crystals comprising niclosamide and a co-former selected from the group consisting of 2-aminothiazole (AT), acetamide (AA), benzamide (BA), and isoniacide (IN).

For the purpose of the specification, a co-crystal shall be regarded as any crystalline structure composed of the two components niclosamide and co-former. More preferably, the co-crystal consists of the two components that form a unique crystalline structure having unique properties.

For the purpose of specification, niclosamide is 2',5-dichloro-4'-nitrosalicylanilide, 5-chloro-*N*-(2-chloro-4-nitrophenyl)-2-hydroxybenzamide, NCL):

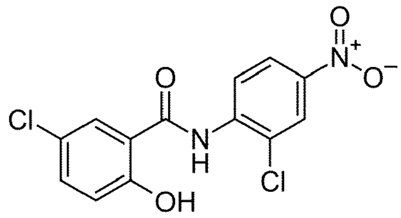

The crystal structures of NCL-AA-I/II, NCL-AT and the metastable monohydrate NCL-$H_A$ were solved from white microcrystalline powder samples based on the established "NMR crystallography" protocol (that is the combined application of solid-state NMR, powder X-ray diffraction and DFT chemical shift computation; see D. Lüdeker et al., Solid State Nucl. Magn. Reson. 2015, 65, 29-40).

Finally, quite significant improvement of the equilibrium solubility of the (1:1) co-crystal NCL-AT could be determined (2.8x that of pristine NCL and 1.4x that of NCL-URE co-crystal), rendering in particular NCL-AT a highly promising candidate for future medical treatment. In addition to solubility improvement of NCL-AT the stability of the co-crystal is improved especially concerning its hygroscopic behavior.

The reliability of hydrogen-bond mediated co-crystal formation for the improvement of the solubility of niclosamide (NCL) considering suitable co-former molecules that contain either amide groups and/or aromatic nitrogen atoms, respectively, was explored. From solvent assisted solid grinding and/or slow solvent evaporation new co-crystals of (i) niclosamide 2-aminothiazole (NCL-AT), (ii) niclosamide - benzamide (NCL-BA), (iii) niclosamide - isoniazide (NCL-IN) and the polymorphic system (iv) niclosamide - acetamide I and II (NCL-AA-I/ NCL-AA-II) were successfully prepared.

In order to enhance the probability of successful co-crystal formation of NCL all selected co-former molecules offer a competitive hydrogen-bond acceptor such as other C=O units or aromatic nitrogen atoms (Figure 1) that upon solvent assisted solid grinding and/or slow solvent evaporation typically yielded micro-crystalline powdered samples rather than single crystals.

Preferably, the relative molar ratio of the niclosamide to the co-former is within the range of from about 5:1 to about 1:5, more preferably about 4:1 to about 1:4, still more preferably about 3:1 to about 1:3, yet more preferably about 2:1 to about 1:2, even more preferably about 1.5:1 to about 1:1.5, most preferably about 1.1:1 to about 1:1.1, and in particular about 1:1.

Preferably, the co-crystals according to the invention essentially consist of niclosamide and co-former, i.e. essentially do not contain any ingredients other than niclosamide and co-former.

In a preferred embodiment of the invention, the co-former is 2-aminothiazole (NCL-AT).

Preferably the co-crystal

- has one or more characteristic XRPD diffraction peaks at 20 ($\pm$0.2) under ambient conditions at Cu K$\alpha$ radiation selected from the group consisting of 7.0°, 9.4°, 15.8°, 26.4° and 27.6°; and/or
- is in the monoclinic crystal system, preferably space group $P2_1/c$; and/or
- has a melting point of about 187.5°C ($\pm$2.0); and/or
- has a solubility at 37°C (24 h) in 40% isopropanol-water mixture of about 246 mg $L^{-1}$.

In another preferred embodiment of the invention, the co-former is acetamide (NCL-AA).

Preferably, the co-crystal comprises or is polymorph I (NCL-AA-I) which

- has one or more characteristic XRPD diffraction peaks at 2Θ ($\pm$0.2) under ambient conditions at Cu K$\alpha$ radiation selected from the group consisting of 8.7°, 14.4°, 18.7°, 25.9°, and 26.6°; and/or
- is in the triclinic crystal system, preferably space group $P\overline{1}$; and/or
- has a melting point of about 159.2°C ($\pm$2.0); and/or
- has a solubility at 37°C (24 h) in 40% isopropanol-water mixture of about 122 mg $L^{-1}$; and/or polymorph II (NCL-AA-II) which

- has one or more characteristic XRPD diffraction peaks at 20 ($\pm$0.2) under ambient conditions at Cu K$\alpha$ radiation selected from the group consisting of 6.1°, 8.8°, 17.7°, 19.6° and 26.5°; and/or

- is in the orthorhombic crystal system, preferably space group $P2_12_12_1$; and/or
- has a melting point of about 157.7°C (±2.0).

In still another preferred embodiment of the invention, the co-former is benzamide (NCL-BA).
Preferably, the co-crystal

- has one or more characteristic XRPD diffraction peaks at 2θ (±0.2) under ambient conditions at Cu Kα radiation selected from the group consisting of 5.6°. 8.0°, 12.2°, 16.7°, and 25.6°; and/or
- has a melting point of about 189.6°C (±2.0); and/or
- has a solubility at 37°C (24 h) in 40% isopropanol-water mixture of about 109 mg $L^{-1}$.

In yet another preferred embodiment of the invention, the co-former is isoniacide (NCL-IN).
Preferably, the co-crystal

- has one or more characteristic XRPD diffraction peaks at 2θ (±0.2) under ambient conditions at Cu Kα radiation selected from the group consisting of 5.1°, 5.6°, 7.6°, 24.0° and 27.3°; and/or
- has a melting point of about 185.5°C (±2.0); and/or
- has a solubility at 37°C (24 h) in 40% isopropanol-water mixture of about 105 mg $L^{-1}$.

Another aspect of the invention relates to a pharmaceutical composition comprising one or more co-crystals according to the invention. All preferred embodiments of the co-crystals according to the invention that have been described above also analogously apply to the pharmaceutical composition according to the invention and thus are not repeated hereinafter.
Preferably, essentially the total amount of niclosamide that is contained in the pharmaceutical composition as well as essentially the total amount of the co-former that is contained in the pharmaceutical composition is contained in said one or more co-crystals according to the invention.
A "pharmaceutical composition" as referred to in the present application comprises at least one co-crystal according to the invention and at least one pharmaceutically acceptable carrier.
For preparing pharmaceutical compositions from the co-crystals according to the invention, pharmaceutically acceptable carriers can be either solid or liquid. Solid pharmaceutical compositions include powders, tablets, pills, capsules, cachets, suppositories, and dispersible granules. A solid carrier can be one or more substances, which may also act as diluents, flavoring agents, binders, preservatives, tablet disintegrating agents, or an encapsulating material.
In powders, the carrier is preferably a finely divided solid, which is preferably in a mixture with the finely divided active component. In tablets, the co-crystals are mixed with the carrier having the necessary binding properties in suitable proportions and compacted in the shape and size desired.
The powders and tablets preferably contain from 5% to 80%, more preferably from 20% to 70% of the co-crystals. Suitable carriers include but are not limited to magnesium carbonate, magnesium stearate, talc, sugar, lactose, pectin, dextrin, starch, gelatin, tragacanth, methylcellulose, sodium carboxymethylcellulose, a low melting wax, cocoa butter, and the like.
Another aspect of the invention relates to a pharmaceutical dosage form comprising the pharmaceutical composition according to the invention as described above. All preferred embodiments of the pharmaceutical composition according to the invention that have been described above also analogously apply to the pharmaceutical dosage form according to the invention and thus are not repeated hereinafter.
Preferably, the pharmaceutical dosage form according to the invention is for oral administration.
The term "dosage form" is intended to include the formulation of the co-crystal with encapsulating material as a carrier providing a capsule in which the co-crystal with or without other carriers, is surrounded by a carrier, which is thus in association with it. Similarly, cachets and lozenges are included. Tablets, powders, capsules, pills, cachets, and lozenges can be used as solid dosage forms suitable for oral administration.
For preparing suppositories, a low melting wax, such as a mixture of fatty acid glycerides or cocoa butter, is first melted and the co-crystals are dispersed homogeneously therein, as by stirring. The molten homogeneous mixture is then poured into convenient sized molds, allowed to cool, and thereby to solidify.
Liquid dosage forms include solutions, suspensions, and emulsions, for example, water or water/propylene glycol solutions. For parenteral injection, liquid dosage forms can be formulated in solution in aqueous polyethylene glycol solution, provided that the co-crystals remain crystalline.
Liquid dosage forms suitable for oral use can be prepared by suspending the co-crystals in a suitable solvent

and adding suitable colorants, flavors, stabilizers, and thickening agents as desired. Suspensions suitable for oral use can be made by dispersing the finely divided co-crystals in a suitable solvent with viscous material, such as natural or synthetic gums, resins, methylcellulose, sodium carboxymethylcellulose, and other well-known suspending agents.

Also included are solid dosage forms, which are intended to be converted, shortly before use, to liquid form preparations for oral administration. Such liquid forms include solutions, suspensions, and emulsions. These preparations may contain, in addition to the co-crystals, colorants, flavors, stabilizers, buffers, artificial and natural sweeteners, dispersants, thickeners, solubilizing agents, and the like.

The pharmaceutical dosage form is preferably in unit dosage form. In such form the composition is subdivided into unit doses containing appropriate quantities of the co-crystals. The dosage form can be a packaged preparation, the package containing discrete quantities of preparation, such as packeted tablets, capsules, and powders in vials or ampoules. Also, the unit dosage form can a capsule, tablet, cachet, or lozenge itself, or it can be the appropriate number of any of these in packaged form.

The co-crystals, pharmaceutical compositions and pharmaceutical dosage forms according to the invention can be administered by various well known routes, including oral, rectal, intragastrical, intracranial and parenteral administration, e.g. intravenous, intramuscular, intranasal, intradermal, subcutaneous, and similar administration routes. Parenteral administration and particular intravenous administration, preferably by depot injection, is preferred. Depending on the route of administration different pharmaceutical formulations are required and some of those may require that protective coatings are applied to the drug formulation to prevent degradation of a compound of the invention in, for example, the digestive tract.

Thus, preferably, the co-crystals of the invention is formulated as a syrup, an infusion or injection solution, a tablet, a capsule, a capslet, lozenge, a liposome, a suppository, a plaster, a band-aid, a retard capsule, a powder, or a slow release formulation. Preferably the diluent is water, a buffer, a buffered salt solution or a salt solution and the carrier preferably is selected from the group consisting of cocoa butter and vitebesole.

Particular preferred pharmaceutical dosage forms for the administration of a compound of the invention are forms suitable for injectable use and include sterile aqueous solutions or dispersions and sterile powders for the extemporaneous preparation of sterile injectable solutions or dispersion. In all cases the final solution or dispersion form must be sterile and fluid. Typically, such a solution or dispersion will include a solvent or dispersion medium, containing, for example, water-buffered aqueous solutions, e.g. biocompatible buffers, ethanol, polyol, such as glycerol, propylene glycol, polyethylene glycol, suitable mixtures thereof, surfactants or vegetable oils. A compound of the invention can also be formulated into liposomes, in particular for parenteral administration. Liposomes provide the advantage of increased half life in the circulation, if compared to the free drug and a prolonged more even release of the enclosed drug.

Sterilization of infusion or injection solutions can be accomplished by any number of art recognized techniques including but not limited to addition of preservatives like anti-bacterial or anti-fungal agents, e.g. parabene, chlorobutanol, phenol, sorbic acid or thimersal. Further, isotonic agents, such as sugars or salts, in particular sodium chloride may be incorporated in infusion or injection solutions.

Production of sterile injectable solutions containing one or several of the compounds of the invention is accomplished by incorporating the respective compound in the required amount in the appropriate solvent with various ingredients enumerated above as required followed by sterilization. To obtain a sterile powder the above solutions are vacuum-dried or freeze-dried as necessary diluents of the present invention are water, physiological acceptable buffers, physiological acceptable buffer salt solutions or salt solutions. Preferred carriers are cocoa butter and vitebesole. Besides the preferred excipients mentioned already above, also the following excipients can be chosen, without limitation, to be used with the various pharmaceutical dosage forms of a compound of the invention:

a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like;

b) lubricants such as magnesium stearate, talc, calcium stearate, zinc stearate, stearic acid, hydrogenated vegetable oil, leucine, glyceride and sodium stearyl fumarates,

c) disintegrants such as starches, croscaramellose, sodium methyl cellulose, agar, bentonite, alginic acid, carboxymethyl cellulose, polyvinyl pyrrolidone and the like.

Other suitable excipients can be found in the Handbook of Pharmaceutical Excipients, published by the American Pharmaceutical Association, which is herein incorporated by reference.

It is to be understood that depending on the severity of the disorder and the particular type which is treatable with one of the compounds of the invention, as well as on the respective patient to be treated, e.g. the general health status of the patient, etc., different doses of the respective compound are required to elicit a therapeutic or prophylactic effect. The determination of the appropriate dose lies within the discretion of the attending physician.

As is known in the art, the pharmaceutically effective amount of a given composition will also depend on the administration route. In general the required amount will be higher, if the administration is through the gastrointestinal tract; e.g. by suppository, rectal, or by an intragastric probe, and lower if the route of administration is parenteral, e.g. intravenous.

Preferably, the co-crystals are formulated for oral, intravenous, intrathecal intraparenchymal administration.

Administration frequencies are not particularly limited. Preferably, administration proceeds once daily (sid), twice daily (bid) or thrice daily (tid).

Another aspect of the invention relates to a co-crystal according to the invention, a pharmaceutical composition according to the invention, or a pharmaceutical dosage form according to the invention, for use in the prevention or treatment of inflammatory diseases (e.g. rheumatoid arthritis), neurodegenerative diseases, or cancer.

Another aspect of the invention relates to the use of niclosamide and a co-former according to the invention for the manufacture of a co-crystal according to the invention, a pharmaceutical composition according to the invention, or a pharmaceutical dosage form according to the invention for the prevention or the treatment of inflammatory diseases (e.g. rheumatoid arthritis), neurodegenerative diseases, or cancer.

Another aspect of the invention relates to a method for the prevention or the treatment of inflammatory diseases (e.g. rheumatoid arthritis), neurodegenerative diseases, or cancer comprising the administration of a therapeutically effective amount of a co-crystal according to the invention, a pharmaceutical composition according to the invention, or a pharmaceutical dosage form according to the invention to a subject in need thereof.

All preferred embodiments of the co-crystal according to the invention, of the pharmaceutical composition according to the invention, or of the pharmaceutical dosage form according to the invention that have been described above also analogously apply to the medical uss according to the invention and thus are not repeated hereinafter.

The following examples further illustrate the invention but are not to be construed as limiting its scope.

*General procedures and results*

*Powder X-ray Diffraction*: Bulk samples for phase identification (and where feasible even for structure solution) were analyzed by PXRD on a Bruker D8 Discover powder diffractometer. Experimental conditions: Cu-K$\alpha_{1/2}$ radiation ($\lambda_1$ = 1.540596 Å, $\lambda_2$ = 1.544410 Å; ratio K$\alpha_1$/ K$\alpha_2$ = 0.441122); 40 kV: 40 mA: scanning interval 5-50° 20 at a step size of 0.01°, time per step 3 s; T = 293 K in Bragg-Bretano geometry. Except for NCL-AT and NCL-AA-I, the samples for structure solution were analyzed with a STOE StadiP powder diffractometer. Experimental conditions: Cu-K$\alpha_1$ radiation $\lambda_1$ = 1.540598 Å); 45 kV; 30 mA; scanning interval 0-70° 20 at a step size of 0.10; time per step 60 s; T = 293 K in Debye-Scherrer geometry. Indexing of the powder patterns was performed using N-TREOR and further confirmed with DICVOL04. The corresponding cell volume was verified by calculating expected cell volumes from volume increments. For space group determination Pawley refinement was used for whole pattern fitting thereby extracting the integrated peak intensities and their correlations using the expo2014 software package. Structures were solved in direct space using the simulated annealing (SA) approach or hybrid big bang big crunch (HBB-BC) algorithm as implemented in expo2014, allowing flexible torsion angles as well as six degrees of freedom for rotation and translation, respectively, for each molecule. After an initial structure model was obtained with all hydrogen atoms removed during the optimization (50 runs) hydrogen atoms were inserted at typical positions using Mercury 3.3 and Rietveld refinement was then performed using Bruker TOPAS 4.2.

The synthesis of novel co-phases was independently checked based on the recorded powder X-ray diffraction pattern and subsequent comparison of the PXRD pattern of both the starting compounds and obtained products thereby documenting the successful co-crystal formation for all considered co-formers (Figure 1): NCL-AA, NCL-AT, NCL-BA, NCL-IN as well as the reproduced co-crystals NCL-NA and NCL-IA. In addition the metastable monohydrate (NCL-$H_A$) could be isolated in its pristine form. Note that in all cases attempts to grow larger crystals suitable for single-crystal XRD analysis from slow solvent evaporation were not successful. For this reason the de-novo structure solution from powder XRD data was performed taking the complementary structural input from available solid state NMR data (particularly the content of the asymmetric unit and selective hydrogen-bond formation) of the respective co-phases into account ("NMR crystallography").

An inspection of the PXRD pattern of the starting compound NCL anhydrate revealed that its most intense reflections are at 20 = {13.3°, 13.9°, 26.1°. 26.7° and 27.2°} so that changes of those peaks were indicative of co-crystal formation similarly to the $^1$H MAS NMR data. For NCL-$H_A$ the characteristic reflections are at 20 = {9.4°, 10.6°, 16.9°, 25.6° and 27.2°} while major reflections at 2 $\Theta$ = {10.4°, 13.1°, 22.4°, 26.8° and 27.3°} are identified in the theoretical XRD pattern computed from available single crystal structure data of NCL-$H_B$. Note that all three XRD pattern are significantly different from each other as expected in case of three independent co-phases (Supporting Information).

Likewise, the most significant new reflections for NCL-AT are at 20 = {7.0°, 9.4°, 15.8°, 26.4° and 27.6°} for NCL-AA-I at 20 = {8.7°. 14.5°, 18.7°, 25.9°, and 26.6°} and for NCL-AA-II at 2 $\Theta$ = {6.1°, 8.8°, 17.7°, 19.6° and 26.5°}, respectively.

Also, the co-crystals NCL-BA and NCL-IN yielded characteristic reflections at 2θ = {5.6°, 8.0°, 12.2°, 16.7°, and 25.6°} and at 2Θ = {5.1°, 5.6°, 7.6°, 24.0° and 27.3°}.

Finally, the two known co-crystals NCL-IA and NCL-NA have been reproduced for further structural characterization via the "NMR crystallography" protocol since no explicit structure solutions were provided in the original work. Their most significant reflections are at 2θ = {5.4°, 7.7°, 13.9°, 27.0°, and 27.6°} (NCL-IA) and 2θ = {5.5°, 7.7°, 14.0°, 27.1°, and 27.6°} (NCL-NA; Figure 3).

The explicit structure solution in all considered cases started with profile fitting and indexing of the recorded PXRD pattern with both N-TREOR and DICVOL04 as implemented in the expo2014 and DASH software packages until reasonable solutions were obtained. The indexing attempts for the co-crystals NCL-NA. NCL-IA, NCL-BA and NCL-IN yielded rather flat unit cells that were similar to the unit cells of NCL-$H_A$, NCL-AA-II and NCL-MeOH, respectively.

Table 1: Crystallographic parameters obtained from the crystal structures of NCL-$H_A$ and the (1:1) co-crystals of NCL with 2-aminothiazole (AT) and acetamide (AA), respectively, as solved by "NMR crystallography"

| | NCL-AT | NCL-AA-I | NCL-AA-II | NCL-$H_A$ (ref.) |
|---|---|---|---|---|
| emp. formula | $C_{16}H_{12}Cl_2N_4O_4S$ | $C_{15}H_{13}Cl_2N_3O_5$ | $C_{15}H_{13}Cl_2N_3O_5$ | $C_{13}H_{10}Cl_2N_2O_5$ |
| formula wt. | 427.26 | 386.19 | 386.19 | 345.13 |
| crystal system | monoclinic | triclinic | orthorhombic | monoclinic |
| space group | $P2_1/c$ | P-1 | $P2_12_12_1$ | $P2_1/a$ |
| T/K | 295 | 295 | 295 | 295 |
| a/Å | 12.8993(3) | 11.2104(4) | 20.0009(9) | 23.0546(5) |
| b/Å | 18.7789(2) | 11.0195(3) | 21.2212(2) | 16.1530(3) |
| c/Å | 7.4498(2) | 7.5701(2) | 3.8816(2) | 3.8120(8) |
| $\alpha$/° | 90 | 99.524(3) | 90 | 90 |
| $\beta$/° | 99.649(3) | 103.185(2) | 90 | 92.824(2) |
| $\gamma$/° | 90 | 108.384(2) | 90 | 90 |
| volume/Å$^3$ | 1779.08(8) | 835.02(5) | 1647.55(2) | 1417.88(5) |
| Z | 4 | 2 | 4 | 4 |
| Z' | 1 | 1 | 1 | 1 |
| radiation type $\lambda$/Å | 1.540596 | 1.540596 | 1.540598 | 1.540598 |
| $R_{exp}$ | 2.586 | 1.771 | 1.395 | 1.132 |
| $R_{wp}$ | 4.227 | 6.351 | 6.235 | 1.977 |
| $R_p$ | 3.327 | 4.155 | 4.431 | 1.415 |
| GOF | 1.634 | 3.587 | 4.470 | 1.747 |
| $\chi^2$ | 2.670 | 12.867 | 19.981 | 3.052 |
| diffractometer | Bruker D8 Discover | Bruker D8 Discover | STOE StadiP | STOE StadiP |

*Solid-State NMR spectroscopy*: Solid-state NMR measurements were performed on either the BRUKER Avance III 300 or Avance DSX 500, corresponding to magnetic flux densities of 7.05 T and 11.6 T, respectively. The spectrometers were equipped with commercially available BRUKER 4 mm double and triple resonance probes operating at MAS rotation frequencies between 3.0 and 15.0 kHz while at 500 MHz spectrometer 2.5 mm triple and double resonance probes operating at MAS rotation frequencies of up to 30.0 kHz were used. $^{13}C\{^1H\}$ cross-polarization (CP)-MAS NMR spectra were measured with $^1H$ 90° pulse lengths of 5$\mu$s (corresponding to a radiofrequency field ($v_1$) of 50 kHz), a contact time of 2.5 ms with spinning frequency of 12 kHz and relaxation delays of 10 to 120 s. The Hartmann - Hahn conditions were adjusted on 1-$^{13}C$-$^{15}N$-labelled $\alpha$-glycine. Efficient polarization transfer was achieved by a ramped-amplitude CP step with $v_1(^1H)$ being swept from 54 kHz to 27 kHz in 64 steps (in the case of a $^1H$ 90° pulse length of 5.0 $\mu$s). All the spectra were obtained with TPPM-15 proton decoupling during the data acquisition applying decoupling pulses of 6.7 $\mu$s to 10.0

μs length (~10/12 μ-pulse). Chemical shifts are reported relative to the secondary standard I-$^{13}C$-$^{15}N$-labelled glycine (1-$^{13}C$ signal at 176.5 ppm). The $^{15}N\{1H\}$ CPMAS-NMR experiments were performed at 7.05 T using the following acquisition parameters: a $^{1}H$ 90° pulse length of 7.0 μs, a contact time of 5 ms, a spinning speed of 10 kHz and a relaxation delay of 10-30 s; $^{15}N$ chemical shifts are reported with respect to 1-$^{13}C$-15 N-labelled glycine (set to -347.6 ppm). $^{1}H$ one pulse MAS NMR spectra were measured at 11.6 T with 2.5 μs 90° pulse length, a spinning speed of 29.762 Hz, a dwell time of 16.8 μs and relaxation delays of 120-2000 s. The back-to-back (BaBa) recoupling sequence was used to excite and reconvert double-quantum (DQ) coherences at 11.74 T with 2.5 μs 90° pulse length, a spinning speed of 29.762 Hz, a dwell time of 5 μs and an excitation time of $\tau_{exc}$=33.6 μs. For the 2D $^{1}H$-$^{1}H$ DQ-MAS-NMR experiments 64-256 $t_1$ increments in steps of 33.6 μs and 16 transients per increment were acquired. Further details are given in the figure captions of the respective 2D spectra. Spectral deconvolution and line shape analysis were performed using the DMFIT Software (version 2011).

Following the established protocol of "NMR crystallography" (D. Lüdeker et al., Solid State Nucl. Magn. Reson. 2015, 65, 29-40), the successful formation of co-phases was monitored by both PXRD and solid-state NMR data since structural changes based on hydrogen-bond mediated co-phase formation are immediately reflected by different hydrogen bonding pattern due to coordination of the exposed O-H unit of NCL to a co-former resulting in changes of the corresponding $^{1}H$ magic angle spinning (MAS) NMR spectra (Figure 2), even in those cases where possibly amorphous products would be obtained. In addition based on $^{13}C$ {$^{1}H$} or $^{15}N$\{$^{1}H$\} cross-polarization MAS NMR spectra, a determination of the integrand unit, i.e. the content of the asymmetric unit, which is an essential information with respect to structure solution from PXRD data, is feasible.

An inspection of the $^{1}H$ MAS NMR spectra of NCL and its cocrystals reveals that the $^{1}H$ chemical shift of the NH group of NCL is in the range of {10.3 - 11.7 ppm}, which clearly indicates that the planar conformation including the intramolecular S(6) hydrogen bonded ring of the NCL anhydrate structure (β-conformation) remains intact in all obtained co-crystals. This finding was further verified based on the corresponding $^{15}N$ chemical shift of {245.5 - 248.9 ppm}, in agreement with quite negligible changes of local electron density distributions. It is apparent that the exposed O-H group of NCL is the key for hydrogen-bond mediated co-crystal synthesis and its $^{1}H$ chemical shift shows a strong dependence on the actually formed synthon. The $^{1}H$ chemical shift of cocrystals that contain {O-H···O} hydrogen bonds is at 12 ± 0.5 ppm, which is likely the case for the NCL-BA co-crystal whose crystal structure could not be solved from PXRD data so far. The co-crystal NCL-AT is stabilized by rather strong {O-H···N} hydrogen bonds as reflected by a high $^{1}H$ chemical shift of 15.3 ppm for the NCL-OH group. Based on the observed $^{1}H$ chemical shift of the co-crystals that contain aromatic nitrogen acceptors (including NCL-IN, NCL-NA and NCL-IA) whose crystal structures are not yet plausibly solved from the available PXRD pattern, it indeed can be concluded that the hydroxyl group of NCL coordinates the aromatic nitrogen rather than amide or hydrazine groups of the co-former yielding stronger hydrogen bonds. Note that the integrated area ratio of the peaks after deconvolution of the $^{1}H$ MAS NMR spectra provided a (1:1) stoichiometry of NCL and the co-former in all considered co-crystals including the monohydrate HA, in perfect agreement with the expectation derived from peak counting of the corresponding $^{13}C$ {$^{1}H$} and $^{15}N$-{$^{1}H$} CPMAS NMR spectra.

*Thermal Analysis*: DSC analysis of the co-phases was performed on a NETZSCH DSC 204 Phoenix®. Samples were placed in crimped but vented aluminum pans. A typical sample size was 4 - 10 mg, the measured temperature range was {20 - 250°C} at 10 °C/min; all samples were purged with a dry nitrogen flow at 125 mL/min during the measurement.

The thermal stability and phase transition of drugs or active pharmaceutical ingredients are highly important to elucidate physicochemical and/or pharmacokinetic properties and likely changes thereof upon prolonged storage due to e.g. de-/rehydration or polymorphism, respectively. The DSC curve obtained for NCL-AA-II indicated a second endotherm peak at 171.5°C prior to melting at 157.7°C which is followed by the sublimation of AA and melting process of pristine NCL. In contrast, the polymorph NCL-AA-I showed merely one endotherm peak at 159.2°C in addition to sublimation of AA and melting of NCL. Note that the dehydration of NCL-$H_A$ was observed at 86.9°C while the remaining new co-crystals exhibited a single endotherm peak at temperatures ranging between those of the pristine drug and co-formers thereby suggesting no decomposition before melting (Table 2):

| | Co-former | Phase | mp/°C |
|---|---|---|---|
| reference | - | NCL | 228.3 |
| | water | NCL-$H_A$ | 86.9 (dehydration) |
| | nicotinamide | NCL-NA | 187.6; 202.9; 222.7 |
| | isonicotinamide | NCL-IA | 226.7 |
| | imidazole | NCL-IMI | 213.5 |
| inventive | 2-aminothiazole | NCL-AT | 187.5 |
| | acetamide | NCL-AA-I | 159.2 |
| | acetamide | NCL-AA-II | 157.7; 171.5 |
| | benzamide | NCL-BA | 189.6 |
| | isoniazide | NCL-IN | 185.5 |

Table 2: Melting points and endotherm peaks identified in the corresponding DSC curves of NCL co-crystals and NCL-$H_A$ hydrate

*Solubility Experiments*: The quantification of NCL was validated using a Phenomenex Gemini-NX HPLC-UV/vis and a C18 column (250 mm x 4.6 mm, 5 mm particle diameter). The mobile phase consisted of a 0.05 M MeOH/$(NH_4)_3PO_4$ (9:1) buffer, adjusted to a pH value of 3.6 with $H_3PO_4$. The considered concentration range of known NCL was in between 0.5-100 μg/mL. The flow rate and the injection volume was set to 1.0 mL/min and 50 μL, respectively. The limit of detection was 0.1 μg/mL and the limit of quantification 0.5 μg/mL. The absorbance was measured at 254 nm. The equilibrium solubility was determined in 40% isopropanol-water medium using a shake-flask method. Accordingly, each solid sample containing 50 mg of NCL was stirred for 24 h in 2.5 mL of the medium at 37°C, and the absorbance was measured at 337 nm. The concentration of the saturated solution was calculated after 24 h which is referred to as the equilibrium solubility of the stable solid form.

The corresponding equilibrium solubility of NCL, its novel co-crystals and some reference compounds was determined following established procedures. Notably, the reported solubility values of the NCL-NA, NCL-IA, NCL-URE and NCL-PABA co-crystals differ from the measured values in accordance with the invention, which may result from the fact that in reference P. Sanphui et al., Crystal Growth & Design 2012, 12, 4588-4599 the authors did not normalize the weights of solid material used for solubility tests to the amount of NCL. Co-crystals that contain a co-former with a low weight therefore may exhibit a higher solubility compared to heavier co-crystal formulations. In order to allow for a better comparability of the determined solubility values, the amount of NCL actually present in the tested co-crystals was normalized to 50 mg per sample, therefore adjusting the initially weighted sample accordingly. The best solubility improvement could be achieved in the case of NCL-AT where the equilibrium solubility was 2.8 times higher than that of pristine NCL, which at present corresponds to the highest equilibrium solubility of NCL co-crystals obtained thus far. In addition a moderate increase of the equilibrium solubility was observed for the co-crystals of NCL-BA, NCL-IN and NCL-AA-I, respectively (Table 3). Note that the co-crystals NCL-PABA, NCL-URE and NCL-CAF tend to hydrate formation after a period of 4 to 6 weeks (P. Sanphui et al., Crystal Growth & Design 2012, 12,4588-4599), while the NCL-AT co-crystal is stable for many months, probably due to its robust and strong hydrogen bonds.

| | Phase | Solubility at 37° C (24 h) in 40% isopropanol-water mixture (mg $L^{-1}$) | factor | factor* |
|---|---|---|---|---|
| reference | NCL | 87.15 | | |
| | NCL-URE | 84.89 | 1.0 | 2.0 |
| | NCL-CAF | 114.5 | 1.3 | 1.3 |
| | NCL-PABA | 109.1 | 1.1 | 0.8 |
| | NCL-NA | 114.45 | 1.3 | 1.2 |
| | NCL-IA | 113.45 | 1.3 | 1.7 |
| inventive | NCL-AT | 245.87 | 2.8 | |
| | NCL-AA-I | 122.29 | 1.4 | |
| | NCL-BA | 109.34 | 1.3 | |
| | NCL-IN | 105.29 | 1.2 | |

Table 3: The determined equilibrium solubility of the considered NCL co-crystals. Reference data obtained from already reported co-crystals (*,P. Sanphui et al., Crystal Growth & Design 2012, 12, 4588-4599) is given in brackets for comparison. Note that the discrepancies likely result from normalization with respect to a fixed amount of NCL per sample.

*DFT calculations:* For the sake of reasonable accuracy at affordable computational costs, the DFT chemical shift calculations were performed in Gaussian09 considering suitably chosen duster models that were extracted from the obtained PXRD structure solutions or where available from single-crystal data. The cluster models were created such that all characteristic hydrogen bonds or close proximities due to packing were satisfied. Though the hydrogen atoms were Rietveld refined in the final step of the structure solution process, all atoms except for hydrogen were "frozen" at given coordinates for H-bond length optimization (opt = ModRedundant) at DFT/PBE1PBE/6-311G(d,p) level of theory; in all considered cases frequency calculations did not reveal imaginary contributions. Subsequently, {$^{1}H$, $^{13}C$ & $^{15}N$} chemical shieldings were computed from optimized cluster models at PBE1PBE/6-311G(d,p) level of theory and "translated" into the corresponding chemical shifts with respect to either benzene ($^{1}H$, $^{13}C$) or methanol ($^{1}H$, $^{13}C$), and glycine ($^{15}N$), respectively, following the multi-standard approach.

The evaluation of the correctness of structure solutions derived from profile fitting and analysis of PXRD pattern may be challenging without complementary data. Therefore, all obtained structure solutions were validated based on experimental solid-state NMR data and $^{1}H$, $^{13}C$ and $^{15}N$ DFT chemical shift computations of representative structure models (representative cutouts from the structure solution) where all important hydrogen bonding pattern and packing features of the co-crystals were taken into account. In case of the co-compounds NCL-BA, NCL-IN, NCL-NA or NCL-IA no acceptable structure solutions were derived from thorough analysis of the respective PXRD pattern that could fulfill the "NMR crystallography" procedure, i.e. the computed DFT NMR shifts of representative "frozen core" cluster cutouts did not match with the experimentally observed chemical shifts within the required accuracy. As a consequence such structure solutions were considered unreliable and hence not further considered.

Note that the calculated shifts fit very well (± 0.5 ppm) with the corresponding experimental values of the presented structure solutions of NCL-$H_A$, NCL-AT, NCL-AA-I and NCL-AA-II, as well as in case of the reported single-crystal structures NCL-CAF and NCL-URE that were included for comparison and further validation of the "NMR crystallography" protocol. This finding clearly emphasizes the validity of this interdisciplinary approach rendering the novel crystal structures obtained from the powdered co-compounds rather reliable. In contrast, the DFT computed chemical shifts of tentative structure solutions derived for the co-compounds NCL-BA, NCL-IN, NCL-NA and NCL-IA, respectively, were inconsistent with the available experimental solid state NMR data so that these structure solutions were considered incorrect and hence discarded even though they exhibited plausible hydrogen bonding pattern as well as reasonably good profile fits and *R*-values.

| | Phase | H-bond motif | $^1$H, $\delta_{iso}$ (DFT)/ppm | $^1$H, $\delta_{iso}$ (exp)/ppm | d(X-H···Y)/ Å | Angle/ ° |
|---|---|---|---|---|---|---|
| reference | NCL-H$_A$ | $_{NCL}$O-H···O | 11.0 | 11.0$^{DQ}$ | 1.742 | 168.6 |
| | | $_{NCL}$N-H···OH$_{NCL}$ | 11.7 | 11.6$^{DQ}$ | 1.717 | 140.5 |
| | | $_{H2O}$O-H···O$_{ket}$ | 5.2 | 4.0$^{DQ}$ | 1.803 | 176.6 |
| | | $_{H2O}$O-H···O$_{nitro}$ | 3.5 | 4.7$^{DQ}$ | 2.063 | 168.1 |
| | NCL-H$_B$ | $_{NCL}$O-H···O | 12.6 | 11.6* | 1.616 | 171.9 |
| | | $_{NCL}$N-H···OH$_{NCL}$ | 11.9 | 11.6* | 1.747 | 140.1 |
| | | $_{H2O}$O-H···O$_{ket}$ | 4.7 | 4.6* | 1.891 | 168.6 |
| | | $_{H2O}$O-H···O$_{nitro}$ | 4.8 | 4.6* | 1.981 | 168.0 |
| | NCL-NA | $_{NCL}$O-H···N$_{arom}$ | n.s. | 13.7 | n.s. | n.s. |
| | | $_{NCL}$N-H···OH$_{NCL}$ | n.s. | 11.0 | n.s. | n.s. |
| | NCL-IA | $_{NCL}$O-H···N$_{arom}$ | n.s. | 14.4 | n.s. | n.s. |
| | | $_{NCL}$N-H···OH$_{NCL}$ | n.s. | 10.7 | n.s. | n.s. |
| | NCL$^-$-IMI$^+$ | $_{NCL}$O$^-$···$^+$HN$_{arom}$ | 17.9 | 18.5/ 18.2* | 1.613 | 157.1 |
| | | $_{NCL}$N-H···OH$_{NCL}$ | 13.7 | 12.7 | 1.739 | 137.6 |
| | | $_{IMI}$N-H···O$_{ket}$ | 11.7 | 12.7 | 2.223 | 132.7 |
| | NCL-IMI | $_{NCL}$O-H···N$_{arom}$ | 14.0 | 14.9 | 1.669 | 163.7 |
| | | $_{NCL}$N-H···OH$_{NCL}$ | 11.4 | 12.7 | 1.797 | 137.8 |
| | | $_{IMI}$N-H···O$_{ket}$ | 10.7 | 12.7 | 2.24 | 132.6 |
| | NCL-MeOH | $_{NCL}$O-H···OH | 13.1 | 12.3 | 1.526 | 173.2 |
| | | $_{NCL}$N-H···OH$_{NCL}$ | 11.9 | 11.7 | 1.724 | 139.7 |
| | NCL-CAF | $_{NCL}$O-H···O | 10.8 | 10.5 | 1.661 | 177.6 |
| | | $_{NCL}$N-H···OH$_{NCL}$ | 11.4 | 11.0 | 1.774 | 139.3 |
| | NCL-URE | $_{NCL}$O-H···O | 12.5 | 12.2 | 1.599 | 177.3 |
| | | $_{NCL}$N-H···OH$_{NCL}$ | 10.5 | 9.8 | 1.792 | 138.5 |
| inventive | NCL-AT | $_{NCL}$O-H···N$_{arom}$ | 15.7 | 15.3 | 1.587 | 170.6 |
| | | $_{NCL}$N-H···OH$_{NCL}$ | 11.7 | 11.2 | 1.747 | 140.7 |
| | | $_{AT}$N-H···O$_{NCL}$ | 7.5 | 7.1$^{DQ}$ | 2.027 | 174.8 |
| | | $_{AT}$N-H···O$_{NCL}$ | 5.4 | 5.7$^{DQ}$ | 2.271 | 160.4 |
| | NCL-AA-I | $_{NCL}$O-H···O | 12.0 | 12.3 | 1.694 | 176.4 |
| | | $_{NCL}$N-H···OH$_{NCL}$ | 11.0 | 11.0 | 1.810 | 139.4 |
| | | $_{AA}$N-H···O$_{ket}$ | 8.4 | 6.2$^{DQ}$ | 1.933 | 161.7 |
| | | $_{AA}$N-H···O$_{nitro}$ | 7.4 | 8.9$^{DQ}$ | 2.161 | 137.4 |
| | NCL-AA-II | $_{NCL}$O-H···O | 11.5 | 11.8 | 1.756 | 175.5 |
| | | $_{NCL}$N-H···OH$_{NCL}$ | 11.4 | 10.6 | 1.780 | 140.5 |
| | | $_{AA}$N-H···O$_{ket}$ | 7.8 | 6.6$^{DQ}$ | 2.050 | 157.0 |
| | | $_{AA}$N-H···O$_{nitro}$ | 8.2 | 7.9$^{DQ}$ | 1.987 | 142.3 |
| | NCL-BA | $_{NCL}$O-H···O | n.s. | 12.5 | n.s. | n.s. |
| | | $_{NCL}$N-H···OH$_{NCL}$ | n.s. | 11.7 | n.s. | n.s. |
| | NCL-IN | $_{NCL}$O-H···N$_{arom}$ | n.s. | 13.5 | n.s. | n.s. |
| | | $_{NCL}$N-H···OH$_{NCL}$ | n.s. | 11.1 | n.s. | n.s. |

Table 4: Experimental and DFT calculated $^1$H chemical shifts of characteristic hydrogen bonds that stabilize the structures of the considered NCL pseudo-polymorphs and co-crystals. The superscript DQ denotes for the extraction of single quantum coherences from $^1$H-$^1$H-DQ-MAS-NMR data. Co-crystals for which no structure solution was successful are

marked with “n.s.” (no structure). *taken from: F. Grifasi et al., Crystal Growth & Design 2015, 15, 1939-1948.

*Chemicals*

NCL was purchased from Cayman Chemical Company (Tallinn, Estonia) while AA, AT, BA and NA were obtained from Acros Organics (Geel, Belgium): IA and IN were delivered from Sigma Aldrich (Saint-Quentin Fallavier, France). All compounds were used without further purification.

Reference Example 1 - Niclosamide hydrate (NCL-$H_A$)

100 mg (0.31 mmol) of niclosamide were refluxed in water (6 mL) for 2 h and the product was cooled to room temperature over 6 h.

The structure of the monohydrate NCL-$H_A$ was solved in the monoclinic unit cell and corresponding space group recently provided from a Pawley fit, though the cell axes a and c were exchanged for better comparability with the pseudo-polymorphic hydrate NCL-$H_B$. The structure solution converged quite quickly with excellent reproducibility within 50 consecutive hybrid big bang big crunch (HBB-BC) runs as implemented in expo2014. The resulting Rietveld refinement plot is shown in Figure 4. The final refinement gave an excellent goodness of fit (GOF = 1.747) and $R_{wp}$ of 1.977%. Therefore, NCL-$H_A$ crystallizes in the monoclinic space group $P2_1/a$ (No. 14) with one molecule niclosamide and one water molecule in the asymmetric unit (Z' = 1).

For NCL-$H_A$ the characteristic reflections are at 2θ = {9.4°, 10.6°, 16.9°, 25.6° and 27.2°} while major reflections at 2 Θ = {10.4°, 13.1°, 22.4°, 26.8° and 27.3°} are identified in the theoretical XRD pattern computed from available single crystal structure data of NCL-$H_B$.

The water molecule is surrounded in a trimeric way by niclosamide through intermolecular {O-H···O} hydrogen bonds, while the hydroxyl group of niclosamide is locked in an almost planar conformation by intramolecular {N-H···O} hydrogen bonding (1.717 Å, 140.5°) resulting in the S(6) ring motif already observed in pure NCL. The NCL-hydroxyl group additionally coordinates to the oxygen of the water molecule ({O-R···O}, 1.742 Å, 168.6°) thereby yielding $C_2^2(8)$ chains in which one hydroxyl group of the crystal water coordinates the amide oxygen of NCL ({O-H···O}, 1.803 Å, 176.6°) as well as $C_2^2(14)$ chains in which the other hydroxyl group of water coordinates the nitro group of NCL (O-H···O, 2.063 Å, 168.1°) (Figure 5). Note that the short *c*-axis of the unit cell evokes π-π stacking of identical layers (at a distance of 3.81 Å) comparable to the known NCL-MeOH solvate co-crytsal in AAA packing fashion. Likewise, the trimeric coordination as observed in NCL-$H_A$ is present in the crystal structure of the thermodynamically stable hydrate NCL-$H_B$. A comparison of the corresponding unit cells of both hydrates (NCL-$H_A$: $P2_1/a$, a = 23.0546(5) Å, b = 16.1530(3) Å, c = 3.8120(8) Å, $\beta$ = 92.824(2)°; $H_B$: $P2_1/c$, a = 11.332(2) Å, b = 16.964(2) Å, c = 7.346(3) Å, β = 98.281(2)°) reveals that the *a*-axis is approximately halved and the *c*-axis is doubled through the transformation from the hydrate $H_A$ to $H_B$. This change in the packing results in much less efficient π-π stacking in $H_B$. It is assumed that the π-π stacking of niclosamide molecules is responsible for the crystallization of the kinetically favored hydrate $H_A$.

Table 5 here below shows the atomic parameters for NCL-$H_A$ without DFT optimization of hydrogen atoms (numeration in accordance with Figure 12):

| atom | *x* | *y* | *z* | occupancy | $U_{iso}$/Å$^2$ |
|---|---|---|---|---|---|
| O5 | 0.35077(16) | 0.34874(29) | -0.0088(18) | 1 | 0.05975 |
| H5A | 0.39504(65) | 0.3545(12) | 0.0157(54) | 1 | 0.07171 |
| H5B | 0.32264(76) | 0.3925(12) | -0.0997(80) | 1 | 0.07171 |
| N1 | 0.27177(14) | 0.60277(20) | 0.64508(87) | 1 | 0.05975 |
| C1 | 0.32725(12) | 0.61073(20) | 0.51594(80) | 1 | 0.05975 |
| C7 | 0.24665(14) | 0.53560(21) | 0.7837(10) | 1 | 0.05975 |
| H1 | 0.24738(67) | 0.6546(13) | 0.6552(61) | 1 | 0.07171 |
| C2 | 0.34514(14) | 0.69035(20) | 0.41239(77) | 1 | 0.05975 |
| C6 | 0.36706(13) | 0.54581(19) | 0.48090(80) | 1 | 0.05975 |

(continued)

| atom | *x* | *y* | *z* | occupancy | $U_{iso}$/Å$^2$ |
|---|---|---|---|---|---|
| O3 | 0.26907(21) | 0.46745(29) | 0.7931(20) | 1 | 0.05975 |
| C8 | 0.18776(12) | 0.54933(20) | 0.9172(10) | 1 | 0.05975 |
| Cl1 | 0.29742(12) | 0.77389(19) | 0.44810(70) | 1 | 0.05975 |
| C3 | 0.39922(13) | 0.70477(20) | 0.28520(72) | 1 | 0.05975 |
| C5 | 0.42172(13) | 0.56032(19) | 0.35185(82) | 1 | 0.05975 |
| H6 | 0.35404(73) | 0.4840(11) | 0.5617(72) | 1 | 0.07171 |
| C13 | 0.15979(13) | 0.62661(20) | 0.95153(83) | 1 | 0.05975 |
| C9 | 0.15903(12) | 0.47778(19) | 1.02214(89) | 1 | 0.05975 |
| C4 | 0.43633(13) | 0.63931(20) | 0.25743(76) | 1 | 0.05975 |
| H3 | 0.41280(72) | 0.7660(10) | 0.2015(51) | 1 | 0.07171 |
| H5 | 0.45267(67) | 0.5102(12) | 0.3280(58) | 1 | 0.07171 |
| O4 | 0.18798(15) | 0.69534(23) | 0.84486(92) | 1 | 0.05975 |
| C12 | 0.10465(13) | 0.63006(19) | 1.08941(75) | 1 | 0.05975 |
| C10 | 0.10377(13) | 0.48348(20) | 1.15894(81) | 1 | 0.05975 |
| H9 | 0.18057(81) | 0.4179(11) | 0.9898(68) | 1 | 0.07171 |
| N2 | 0.49381(14) | 0.65412(21) | 0.12694(94) | 1 | 0.05975 |
| H2 | 0.16625(72) | 0.7450(10) | 0.7690(56) | 1 | 0.07171 |
| C11 | 0.07629(12) | 0.55884(19) | 1.19451(77) | 1 | 0.05975 |
| H12 | 0.08316(70) | 0.6882(12) | 1.1173(49) | 1 | 0.07171 |
| C12 | 0.06697(12) | 0.39501(16) | 1.27768(76) | 1 | 0.05975 |
| O2 | 0.50484(21) | 0.72087(29) | -0.0128(16) | 1 | 0.05975 |
| O1 | 0.52862(21) | 0.59806(32) | 0.1772(19) | 1 | 0.05975 |
| H11 | 0.03420(73) | 0.55980(95) | 1.3170(58) | 1 | 0.07171 |

Reference Example 2 - Niclosamide - nicotinamide (NCL-NA (1:1) co-crystal)

450 mg (1.38 mmol) of niclosamide and 167.99 mg (1.38 mmol) of nicotinamide were ground in mortar-pestle for 15 min after adding 5 drops of dry EtOH. Melting point 222.7 °C (±2.0).
The most significant reflections are at 2θ = {5.5°, 7.7°, 14.0°, 27.1°, and 27.6°} (NCL-NA; Figure 3).

Reference Example 3 - Niclosamide - isonicotinamide (NCL-IA (1:1) co-crystal)

450 mg (1.38 mmol) of niclosamide and 167.99 mg (1.38 mmol) of isonicotinamide were ground in mortar-pestle for 15 min after adding 5 drops of dry EtOH. Melting point 226.7 °C (±2.0).
The most significant reflections are at 2θ = {5.4°, 7.7°, 13.9°, 27.0°, and 27.6°} (NCL-IA).

Example 1 - Niclosamide - 2-aminothiazole (NCL-AT (1:1) co-crystal)

460 mg (1.41 mmol) of niclosamide and 140.82 mg (1.41 mmol) of 2-aminothiazole were ground in mortar-pestle for 15 min after adding 5 drops of dry EtOH. Melting point 187.5 °C (±2.0).
The most significant new reflections for NCL-AT are at 2θ = {7.0°, 9.4°, 15.8°, 26.4° and 27.6°}.
The structure of the (1:1) co-crystal NCL-AT was solved in the monoclinic space group $P2_1/c$ (No. 14) with one molecule of niclosamide and one molecule 2-aminothiazole in the asymmetric unit. Like in the case of NCL-$H_A$ the refinement converged quickly with a very good reproducibility in 50 repeated HBB-BC runs, where the final Rietveld

refinement provided an excellent goodness of fit (GOF = 1.634) and Rwp of 4.227 %. The Rietveld plot is shown in Figure 6.

The intramolecular {N-H···O} hydrogen bond ofNCL (1.747 Å, 140.7°) persists in the (1:1) co-crystal. Notably, 2-Aminothiazole links two NCL molecules via $C_2^2(10)$ chains including a strong intermolecular {O-H···N} hydrogen bond (1.587 Å, 170.6°) of the hydroxyl group of NCL to an aromatic nitrogen of 2-aminothiazole and an intermolecular {N-H···O} hydrogen bond (2.027 Å, 174.8°) connecting the amino group of 2-aminothiazole to the amide oxygen of NCL. The molecules are arranged in ABAB packing fashion along the *c*-axis through π-π stacking (3.645 Å) resulting in a γ-motif (Figure 7).

Table 6 here below shows the atomic parameters for NCL-AT without DFT optimization of hydrogen atoms (numeration in accordance with Figure 13):

| atom | *x* | *y* | *z* | occupancy | $U_{iso}$/ Å$^2$ |
|---|---|---|---|---|---|
| C14 | -0.24156(43) | 0.70519(30) | 0.61467(56) | 1 | 0.02652 |
| S1 | -0.31933(35) | 0.77631(27) | 0.66165(44) | 1 | 0.02652 |
| N4 | -0.28865(49) | 0.64343(30) | 0.58789(47) | 1 | 0.02652 |
| N3 | -0.14025(38) | 0.71961(40) | 0.61139(51) | 1 | 0.02652 |
| C15 | -0.42411(42) | 0.71984(28) | 0.64792(64) | 1 | 0.02652 |
| C16 | -0.39126(40) | 0.65351(27) | 0.60737(69) | 1 | 0.02652 |
| H3A | -0.1021(16) | 0.7238(17) | 0.7083(40) | 1 | 0.03182 |
| H3B | -0.1077(16) | 0.7185(16) | 0.5191(37) | 1 | 0.03182 |
| H15 | -0.4914(23) | 0.7381(18) | 0.6698(38) | 1 | 0.03182 |
| H16 | -0.4402(19) | 0.6136(12) | 0.5921(48) | 1 | 0.03182 |
| N1 | 0.02572(44) | 0.47294(26) | 0.76122(75) | 1 | 0.02652 |
| C1 | 0.12570(34) | 0.48925(29) | 0.86168(59) | 1 | 0.02652 |
| C7 | -0.01105(44) | 0.41159(30) | 0.68895(86) | 1 | 0.02652 |
| H1 | -0.0110(27) | 0.5064(18) | 0.7183(46) | 1 | 0.03182 |
| C2 | 0.14470(40) | 0.56000(29) | 0.92215(67) | 1 | 0.02652 |
| C6 | 0.21074(38) | 0.43928(27) | 0.90704(59) | 1 | 0.02652 |
| O3 | 0.04512(65) | 0.35931(41) | 0.6869(14) | 1 | 0.02652 |
| C8 | -0.12095(33) | 0.41481(28) | 0.58062(64) | 1 | 0.02652 |
| Cl1 | 0.04901(38) | 0.62787(27) | 0.88211(63) | 1 | 0.02652 |
| C3 | 0.23999(37) | 0.58056(26) | 1.02004(62) | 1 | 0.02652 |
| C5 | 0.30626(40) | 0.45983(28) | 1.00601(59) | 1 | 0.02652 |
| H6 | 0.2023(18) | 0.3902(13) | 0.8673(39) | 1 | 0.03182 |
| C13 | -0.19036(38) | 0.47439(27) | 0.55891(67) | 1 | 0.02652 |
| C9 | -0.15702(39) | 0.35070(26) | 0.49217(59) | 1 | 0.02652 |
| C4 | 0.32133(40) | 0.52987(28) | 1.06268(69) | 1 | 0.02652 |
| H3 | 0.2492(19) | 0.6286(13) | 1.0557(45) | 1 | 0.03182 |
| H5 | 0.3607(17) | 0.4247(13) | 1.0338(42) | 1 | 0.03182 |
| O4 | -0.15823(57) | 0.53800(33) | 0.64152(71) | 1 | 0.02652 |
| C12 | -0.29208(35) | 0.46792(27) | 0.45181(67) | 1 | 0.02652 |
| H9 | -0.1114(18) | 0.3098(13) | 0.5076(38) | 1 | 0.03182 |
| C10 | -0.25827(38) | 0.34535(26) | 0.38534(64) | 1 | 0.02652 |

(continued)

| atom | x | y | z | occupancy | $U_{iso}$/ Å$^2$ |
|---|---|---|---|---|---|
| N2 | 0.42446(45) | 0.55227(28) | 1.16935(85) | 1 | 0.02652 |
| H2 | -0.1791(35) | 0.5714(13) | 0.5835(44) | 1 | 0.03182 |
| H12 | -0.3361(15) | 0.5090(17) | 0.4412(48) | 1 | 0.03182 |
| C11 | -0.32768(34) | 0.40253(28) | 0.36191(55) | 1 | 0.02652 |
| C12 | -0.29684(36) | 0.26558(26) | 0.27826(55) | 1 | 0.02652 |
| O2 | 0.44684(73) | 0.61628(37) | 1.1913(15) | 1 | 0.02652 |
| O1 | 0.49065(67) | 0.50469(48) | 1.2054(15) | 1 | 0.02652 |
| H11 | -0.3951(17) | 0.3967(13) | 0.2887(39) | 1 | 0.03182 |

Example 2 - Niclosamide - acetamide

Based on PXRD and solid-state NMR data it was identified that co-crystallization of niclosamide and acetamide in all considered cases yielded polycrystalline mixtures of at least two polymorphs. Using a few drops of ethanol during solvent assisted solid grinding resulted in NCL-AA-I as dominating phase while in case of acetone NCL-AA-II was found as main product. Since no experimental condition could be established that would result in pure co-crystal phases (e.g. only NCL-AA-I or NCL-AA-II) ,multiphase Rietveld refinements were performed after successful structure solutions of-NCL-AA-I and NCL-AA-11 with sufficient reproducibility in the HBB-BC runs (Figure 8; Figure 9). The Rietveld refinement yielded acceptable values for both NCL-AA-I (GOF = 3.587, $R_{wp}$ = 6.351 %) and NCL-AA-II (GOF = 4.470, $R_{wp}$ = 6.235 %), where the fraction of "impurity" through the presence of the other polymorph could be quantified through Rietveld refinement.

In the case of NCL-AA-I, 13.5% NCL-AA-II was found, while the co-crystal of NCL-AA-II contained 6.6% NCL-AA-I. The comparison of the cell parameters of NCL-AA-I and II revealed that both polymorphs are comparable to the case of NCL-$H_A$ and NCL-$H_B$, respectively, though upon transformation of NCL-AA-II into NCL-AA-I the symmetry is lowered from an orthorhombic to a triclinic cell. Therefore, the cell axes a and b are nearly halved whereas the c-axis is doubled so that in total the unit cell content is halved (NCL-AA-I: Z = 2; NCL-AA-II: Z = 4). It appears reasonable to attribute the polymorph NCL-AA-II to a metastable co-crystal form. As previously observed in the pseudo-polymorphic NCL hydrates the $\pi$-$\pi$ stacking in NCL-AA-II (AAA layers) seems to be more efficient than in NCL-AA-I (ABA layers) while the overall crystal packing is more efficient in NCL-AA-I.

*a) NCL-AA-I (1:1) co-crystal*

510 mg (1.56 mmol) of niclosamide and 92.09 mg (1.56 mmol) of acetamide were ground in mortar-pestle for 15 min after adding 5 drops of dry EtOH. Melting point 159.2 °C ($\pm$2.0).

The most significant new reflections for NCL-AA-I are at 2θ = {8.7°. 14.4°, 18.7°, 25.9°, and 26.6°}.

The (1:1) co-crystal NCL-AA-I crystallizes in the triclinic space group $P\overline{1}$ (No. 2) with one molecule of niclosamide and acetamide in the asymmetric unit. Acetamide links two different layers of NCL via a $R_4^4(20)$ ring motif (reminiscent of the NCL - urea co-crystal) through {O-H···O} hydrogen bonds (1.694 Å, 176.4°) of the hydroxyl group of NCL and amide oxygen of acetamide as well as {N-H···O} hydrogen bonds (1.933 Å, 161.7°) of acetamide to the amide oxygen of NCL. Within one layer acetamide links NCL molecules through $C_2^2(12)$ chains over an {N-H···O} hydrogen bond (2.161 Å, 137.4°) to the nitro group and {N-H···O} hydrogen bond to the amide group of NCL, respectively. Niclosamide molecules connect to the next layer by $\pi$-$\pi$ stacking (at a distance of 3.404 Å) along the *c*-axis in ABAB fashion (Figure 10). Table 7 here below shows the atomic parameters for NCL-AA-I without DFT optimization of hydrogen atoms (numeration in accordance with Figure 14):

| atom | x | y | z | occupancy | $U_{iso}$/Å$^2$ |
|---|---|---|---|---|---|
| C14 | 0.89331(78) | 0.95633(88) | -0.2921(21) | 1 | 0.05294 |
| C15 | 1.01377(73) | 1.07586(74) | -0.2129(11) | 1 | 0.05294 |

(continued)

| atom | *x* | *y* | *z* | occupancy | $U_{iso}$/Å$^2$ |
|---|---|---|---|---|---|
| N3 | 0.90305(75) | 0.84728(83) | -0.3600(16) | 1 | 0.05294 |
| O5 | 0.78033(91) | 0.96534(94) | -0.3080(18) | 1 | 0.05294 |
| H2A | 1.0643(25) | 1.0887(25) | -0.3200(31) | 1 | 0.06353 |
| H2B | 0.9836(21) | 1.1578(21) | -0.1769(32) | 1 | 0.06353 |
| H2C | 1.0753(24) | 1.0653(23) | -0.0887(30) | 1 | 0.06353 |
| H3A | 0.8246(22) | 0.7756(23) | -0.4523(37) | 1 | 0.06353 |
| H3B | 0.9920(21) | 0.8430(23) | -0.3376(56) | 1 | 0.06353 |
| N1 | -0.46475(75) | 0.49554(71) | 0.7726(11) | 1 | 0.05294 |
| C1 | -0.41467(73) | 0.40523(75) | 0.84001(90) | 1 | 0.05294 |
| C7 | -0.59600(83) | 0.48249(76) | 0.6908(13) | 1 | 0.05294 |
| H1 | -0.4080(24) | 0.5741(21) | 0.7978(47) | 1 | 0.06353 |
| C2 | -0.27922(74) | 0.44912(72) | 0.91274(86) | 1 | 0.05294 |
| C6 | -0.49606(64) | 0.27675(71) | 0.83888(84) | 1 | 0.05294 |
| C8 | -0.61309(68) | 0.60023(72) | 0.63352(76) | 1 | 0.05294 |
| O3 | -0.6938(10) | 0.38079(92) | 0.6481(18) | 1 | 0.05294 |
| C3 | -0.21716(75) | 0.36972(72) | 0.98790(81) | 1 | 0.05294 |
| Cl1 | -0.17644(59) | 0.60647(57) | 0.91134(72) | 1 | 0.05294 |
| C5 | -0.43994(69) | 0.19214(72) | 0.91189(91) | 1 | 0.05294 |
| H6 | -0.5891(21) | 0.2482(21) | 0.7865(34) | 1 | 0.06353 |
| C13 | -0.51655(64) | 0.72322(74) | 0.65303(85) | 1 | 0.05294 |
| C9 | -0.74364(67) | 0.58451(73) | 0.55096(84) | 1 | 0.05294 |
| C4 | -0.30133(73) | 0.24232(76) | 0.98451(88) | 1 | 0.05294 |
| H3 | -0.1184(22) | 0.4011(22) | 1.0417(34) | 1 | 0.06353 |
| H5 | -0.4928(23) | 0.1022(21) | 0.9144(31) | 1 | 0.06353 |
| C12 | -0.54907(68) | 0.82793(73) | 0.59473(87) | 1 | 0.05294 |
| O4 | -0.38882(81) | 0.74240(87) | 0.7310(10) | 1 | 0.05294 |
| C10 | -0.77751(74) | 0.68890(77) | 0.49043(79) | 1 | 0.05294 |
| H9 | -0.8088(21) | 0.5011(22) | 0.5378(34) | 1 | 0.06353 |
| N2 | -0.24251(78) | 0.15295(77) | 1.0621(12) | 1 | 0.05294 |
| C11 | -0.68148(68) | 0.81369(72) | 0.51070(79) | 1 | 0.05294 |
| H12 | -0.4773(23) | 0.9111(20) | 0.6144(32) | 1 | 0.06353 |
| H2 | -0.3406(25) | 0.8007(22) | 0.7096(34) | 1 | 0.06353 |
| C12 | -0.94460(53) | 0.65443(52) | 0.38718(70) | 1 | 0.05294 |
| O1 | -0.3198(11) | 0.04092(97) | 1.0455(17) | 1 | 0.05294 |
| O2 | -0.12220(98) | 0.1979(10) | 1.1325(19) | 1 | 0.05294 |
| H11 | -0.7024(22) | 0.8833(24) | 0.4737(31) | 1 | 0.06353 |

*b) NCL-AA-II (1:1) Co-crystal*

510 mg (1.56 mmol) of niclosamide and 92.09 mg (1.56 mmol) of acetamide were ground in mortar-pestle for 15 min after adding 5 drops of Acetone. Melting point 157.7 °C (±2.0).

The most significant new reflections for NCL-AA-II are at 2θ = {6.1°, 8.8°, 17.7°, 19.6° and 26.5°}.

The polymorph NCL-AA-II crystallizes in the orthorhombic space group $P2_12_12_1$ (No. 19) with one molecule of niclosamide and acetamide in the asymmetric unit. Acetamide forms infinite columns according to $C_1^1(4)$ chains through {N-H···O} hydrogen bonds (2.050 Å, 157.0°),(Figure 11). The acetamide columns link two crossing layers of NCL along the *c*-axis via $D_2^3(7)$ hydrogen bonding motives at which the hydroxyl group of NCL coordinates the oxygen of acetamide through {O-H···O} hydrogen bonds (1.756 Å, 175.5°). In addition the amide group of AA coordinates the nitro-group of NCL {N-H···O} so that as all donors and acceptors are saturated (1.987 Å, 142.3°). The NCL molecules build two columns that are placed diagonally to each other along the *c*-axis; within the columns the NCL molecules are connected via π-π stacking (3.882 Å) in AAA fashion, similarly to the case of the pseudo-polymorph NCL-$H_A$.

Table 8 here below shows the atomic parameters for NCL-AA-II without DFT optimization of hydrogen atoms (numeration in accordance with Figure 15):

| atom | *x* | *y* | *z* | occupancy | $U_{iso}$/Å$^2$ |
|---|---|---|---|---|---|
| C1 | 0.37687(61) | 0.92463(54) | 0.7769(22) | 1 | 0.05816 |
| C2 | 0.40656(60) | 0.87655(53) | 0.5804(25) | 1 | 0.05816 |
| C3 | 0.37061(62) | 0.82707(46) | 0.4476(30) | 1 | 0.05816 |
| C4 | 0.30229(59) | 0.82665(47) | 0.5066(30) | 1 | 0.05816 |
| C5 | 0.27062(58) | 0.87371(53) | 0.6883(25) | 1 | 0.05816 |
| C6 | 0.30791(60) | 0.92266(55) | 0.8256(27) | 1 | 0.05816 |
| C7 | 0.40389(62) | 1.02941(63) | 1.0316(38) | 1 | 0.05816 |
| C8 | 0.46213(61) | 1.06866(53) | 1.1479(25) | 1 | 0.05816 |
| C9 | 0.44589(60) | 1.12747(55) | 1.2883(22) | 1 | 0.05816 |
| C10 | 0.49552(67) | 1.16842(48) | 1.3960(25) | 1 | 0.05816 |
| C11 | 0.56269(58) | 1.15185(56) | 1.3755(22) | 1 | 0.05816 |
| C12 | 0.57926(61) | 1.09362(54) | 1.2403(24) | 1 | 0.05816 |
| C13 | 0.53010(63) | 1.05237(57) | 1.1217(25) | 1 | 0.05816 |
| C14 | 0.69667(72) | 0.92088(60) | 0.1762(55) | 1 | 0.05816 |
| C15 | 0.65481(59) | 0.86987(58) | 0.1427(35) | 1 | 0.05816 |
| N1 | 0.41923(57) | 0.97108(57) | 0.9093(36) | 1 | 0.05816 |
| N2 | 0.26271(60) | 0.77450(58) | 0.3658(40) | 1 | 0.05816 |
| N3 | 0.76339(64) | 0.91338(56) | 0.3476(47) | 1 | 0.05816 |
| O1 | 0.20227(74) | 0.78055(92) | 0.3476(64) | 1 | 0.05816 |
| O2 | 0.29216(85) | 0.72701(79) | 0.2745(62) | 1 | 0.05816 |
| O3 | 0.34676(67) | 1.04973(87) | 1.0439(73) | 1 | 0.05816 |
| O4 | 0.54774(66) | 0.99592(67) | 0.9828(37) | 1 | 0.05816 |
| O5 | 0.67921(79) | 0.97175(76) | 0.0684(83) | 1 | 0.05816 |
| Cl1 | 0.49148(41) | 0.87942(47) | 0.4931(24) | 1 | 0.05816 |
| Cl2 | 0.47367(45) | 1.24240(44) | 1.5491(28) | 1 | 0.05816 |
| H1 | 0.4686(23) | 0.9596(27) | 0.916(19) | 1 | 0.06979 |

(continued)

| atom | x | y | z | occupancy | $U_{iso}/Å^2$ |
|---|---|---|---|---|---|
| H2 | 0.5956(23) | 0.9842(30) | 0.966(18) | 1 | 0.06979 |
| H3 | 0.3948(26) | 0.7895(27) | 0.299(21) | 1 | 0.06979 |
| H3A | 0.7778(25) | 0.8704(24) | 0.438(18) | 1 | 0.06979 |
| H3B | 0.7945(24) | 0.9512(27) | 0.374(20) | 1 | 0.06979 |
| H5 | 0.2165(24) | 0.8727(26) | 0.724(14) | 1 | 0.06979 |
| H6 | 0.2833(26) | 0.9599(25) | 0.973(22) | 1 | 0.06979 |
| H9 | 0.3936(25) | 1.1413(28) | 1.314(20) | 1 | 0.06979 |
| H11 | 0.6015(26) | 1.1841(28) | 1.467(22) | 1 | 0.06979 |
| H12 | 0.6317(23) | 1.0797(27) | 1.225(16) | 1 | 0.06979 |
| H15A | 0.6425(25) | 0.8512(28) | 0.396(20) | 1 | 0.06979 |
| H15B | 0.6093(22) | 0.8843(26) | 0.014(18) | 1 | 0.06979 |
| H15C | 0.6796(23) | 0.8337(24) | -0.007(22) | 1 | 0.06979 |

Example 3 - Niclosamide - benzamide (NCL-BA (1:1) co-crystal)

450 mg (1.38 mmol) of niclosamide and 166.65 mg (1.38 mmol) of benzamide were ground in mortar-pestle for 15 min after adding 5 drops of dry EtOH. Melting point 189.6 °C (±2.0).

The co-crystals NCL-BA yielded characteristic reflections at 2θ = {5.6°. 8.0°, 12.2°, 16.7°, and 25.6°}.

Example 4 - Niclosamide - isoniazide (NCL-IN (1:1) co-crystal)

430 mg (1.31 mmol) of niclosamide and 180.27 mg (1.31 mmol) of isoniazide were ground in mortar-pestle for 15 min after adding 5 drops of dry EtOH. Melting point 185.5 °C (±2.0).

The co-crystals NCL-IN yielded characteristic reflections at 2θ = {5.1°. 5.6°, 7.6°, 24.0° and 27.3°}.

It becomes clear from the above experimental data that enhanced equilibrium solubility of the crystalline drug niclosamide could be achieved via co-crystal formation based on crystal engineering principles. Suitable co-formers of NCL were taken from the GRAS and EAFUS lists, rendering the resulting co-phases pharmaceutically acceptable and quite promising candidates for the current trend of repurposing long known drugs. Aspects of "green chemistry" were also considered by producing the new crystalline phases from either solvent-drop assisted grinding or mere mechanical treatment in addition to slow evaporation from environment-friendly solvents such as ethanol. In case of NCL-AT, NCL-AA-I/II and NCL-$H_A$, the crystal structures were successfully solved from powdered samples based on the "NMR crystallography" protocol. The hydrogen bonding pattern of pristine NCL was changed by co-crystal formation with an emphasis on particularly robust {O-H···O}, {N-H···O} and {O-H···N} hydrogen bonding motifs.

## Claims

**1.** A co-crystal comprising niclosamide and a co-former selected from the group consisting of 2-aminothiazole, acetamide, benzamide, and isoniacide.

**2.** The co-crystal according to claim 1, wherein

- the relative molar ratio of the niclosamide to the co-former is about 1:1; and/or
- wherein the co-crystals essentially do not contain any ingredients other than niclosamide and co-former.

**3.** The co-crystal according to claim 1 or 2, wherein the co-former is 2-aminothiazole.

**4.** The co-crystal according to claim 3, wherein the co-crystal

- has one or more characteristic XRPD diffraction peaks at 2θ (±0.2) under ambient conditions at Cu Kα radiation selected from the group consisting of 7.0°, 9.4°, 15.8°, 26.4° and 27.6°; and/or
- has a melting point of about 187.5 °C (±2.0); and/or
- has a solubility at 37°C (24 h) in 40% isopropanol-water mixture of about 246 mg $L^{-1}$.

**5.** The co-crystal according to any of claims 1 to 3, wherein the co-former is acetamide.

**6.** The co-crystal according to claim 5, wherein the co-crystal

(I)

- has one or more characteristic XRPD diffraction peaks at 2θ (±0.2) under ambient conditions at Cu Kα radiation selected from the group consisting of 8.7°. 14.4°, 18.7°, 25.9°, and 26.6°; and/or
- has a melting point of about 159.2 °C (±2.0); and/or
- has a solubility at 37°C (24 h) in 40% isopropanol-water mixture of about 122 mg $L^{-1}$; or

(II)

- has one or more characteristic XRPD diffraction peaks at 2θ (±0.2) under ambient conditions at Cu Kα radiation selected from the group consisting of 6.1°, 8.8°, 17.7°, 19.6° and 26.5°; and/or
- has a melting point of about 157.7 °C (±2.0).

**7.** The co-crystal according to any of claims 1 to 3, wherein the co-former is benzamide.

**8.** The co-crystal according to claim 7, wherein the co-crystal

- has one or more characteristic XRPD diffraction peaks at 2θ (±0.2) under ambient conditions at Cu Kα radiation selected from the group consisting of 5.6°. 8.0°, 12.2°, 16.7°, and 25.6°; and/or
- has a melting point of about 189.6 °C (±2.0); and/or
- has a solubility at 37°C (24 h) in 40% isopropanol-water mixture of about 109 mg $L^{-1}$.

**9.** The co-crystal according to any of claims 1 to 3, wherein the co-former is isoniacide.

**10.** The co-crystal according to claim 9, wherein the co-crystal

- has one or more characteristic XRPD diffraction peaks at 2θ (±0.2) under ambient conditions at Cu Kα radiation selected from the group consisting of 5.1°. 5.6°, 7.6°, 24.0° and 27.3°; and/or
- has a melting point of about 185.5 °C (±2.0); and/or
- has a solubility at 37°C (24 h) in 40% isopropanol-water mixture of about 105 mg $L^{-1}$.

**11.** A pharmaceutical composition comprising one or more co-crystals according to any of the preceding claims.

**12.** The pharmaceutical composition according to claim 11, wherein essentially the total amount of niclosamide that is contained in the pharmaceutical composition as well as essentially the total amount of the co-former that is contained in the pharmaceutical composition is contained in said one or more co-crystals.

**13.** A pharmaceutical dosage form comprising a pharmaceutical composition according to claim 11 or 12.

**14.** The pharmaceutical dosage form according to claim 13, which is for oral administration.

**15.** A co-crystal according to any of claims 1 to 10, a pharmaceutical composition according to claim 11 or 12, or a pharmaceutical dosage form according to claim 13 or 14, for use in the prevention or the treatment of inflammatory diseases (e.g. rheumatoid arthritis), neurodegenerative diseases, or cancer.

Figure 1

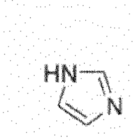
imidazole (IMI)

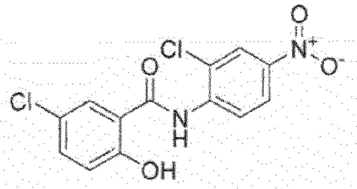
niclosamide (NCL)

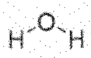
water ($H_A$)

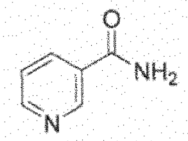
nicotinamide (NA)

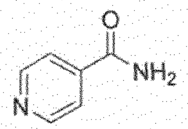
isonicotinamide (IA)

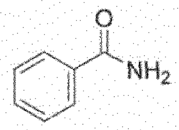
benzamide (BA)

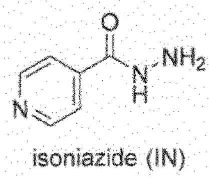
isoniazide (IN)

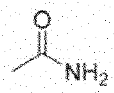
acetamide (AA)

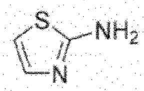
2-aminothiazole (AT)

Figure 2

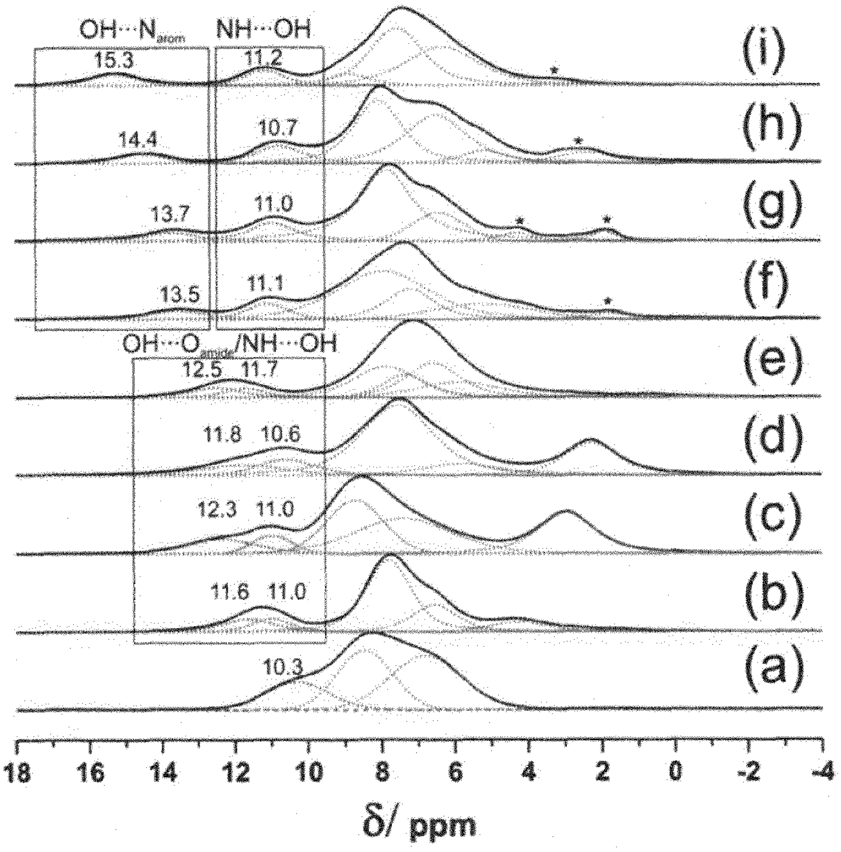

Figure 3

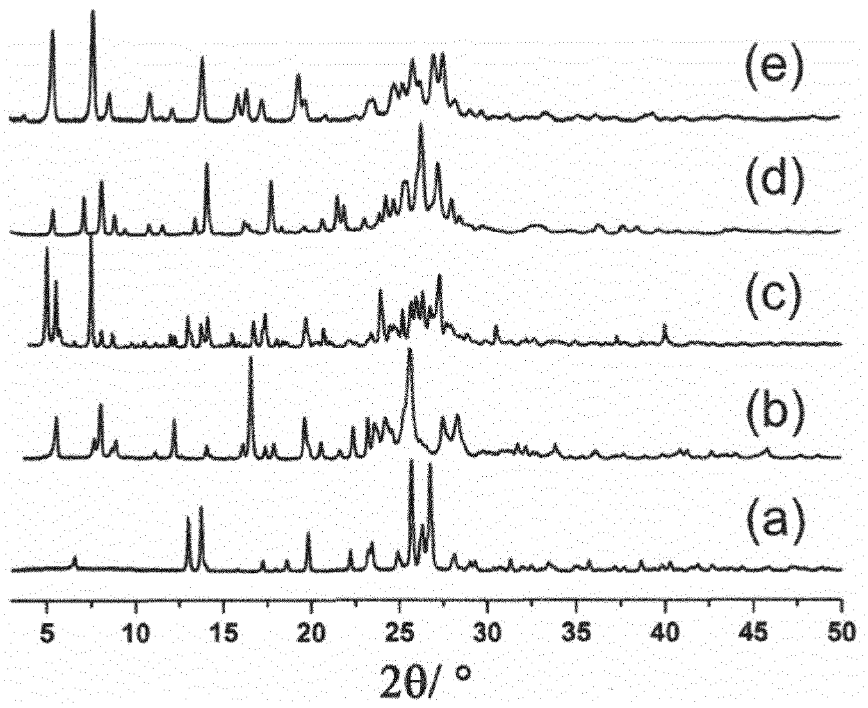


Figure 4

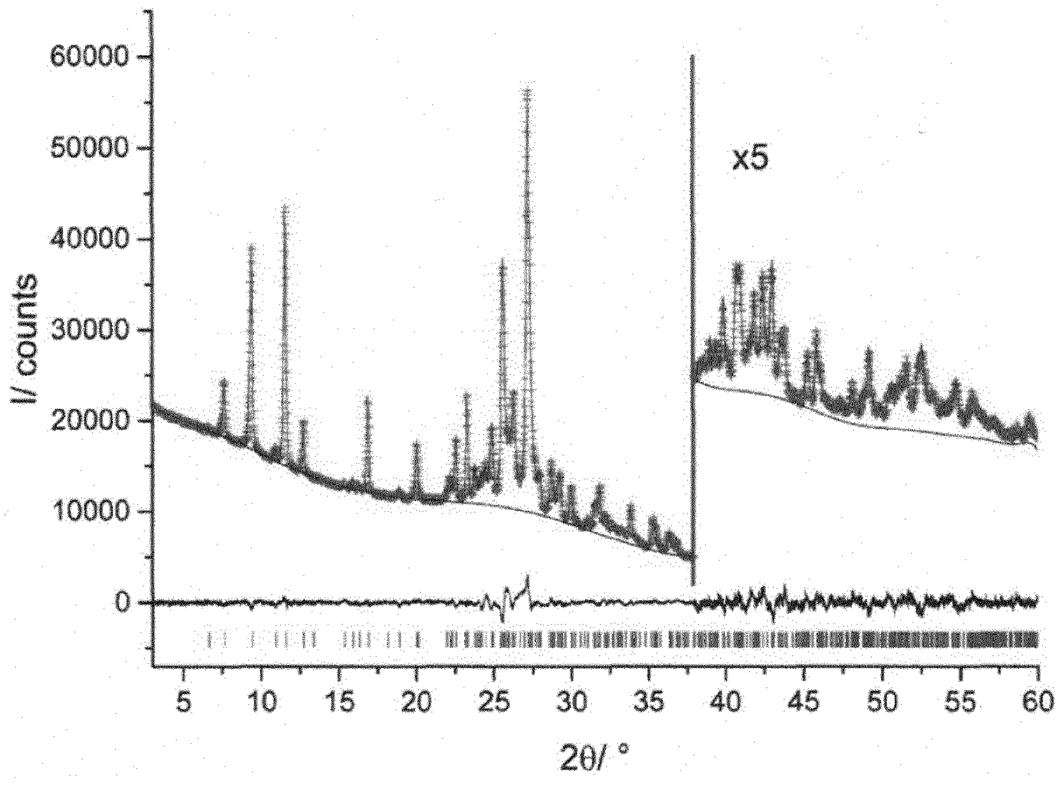

**Figure 5**

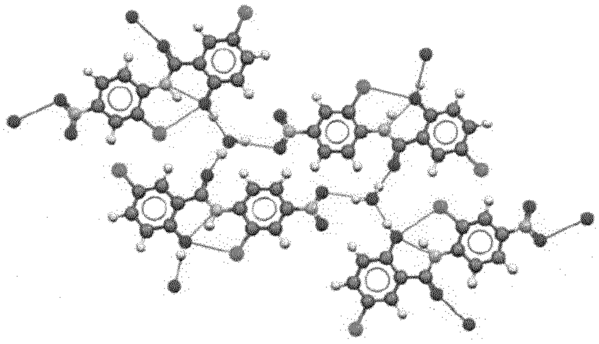

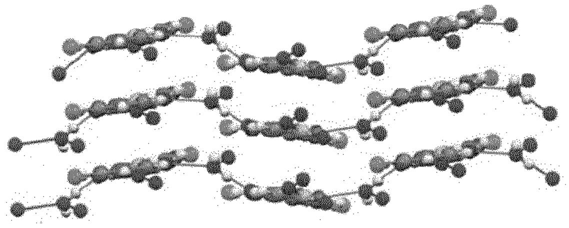

**Figure 6**

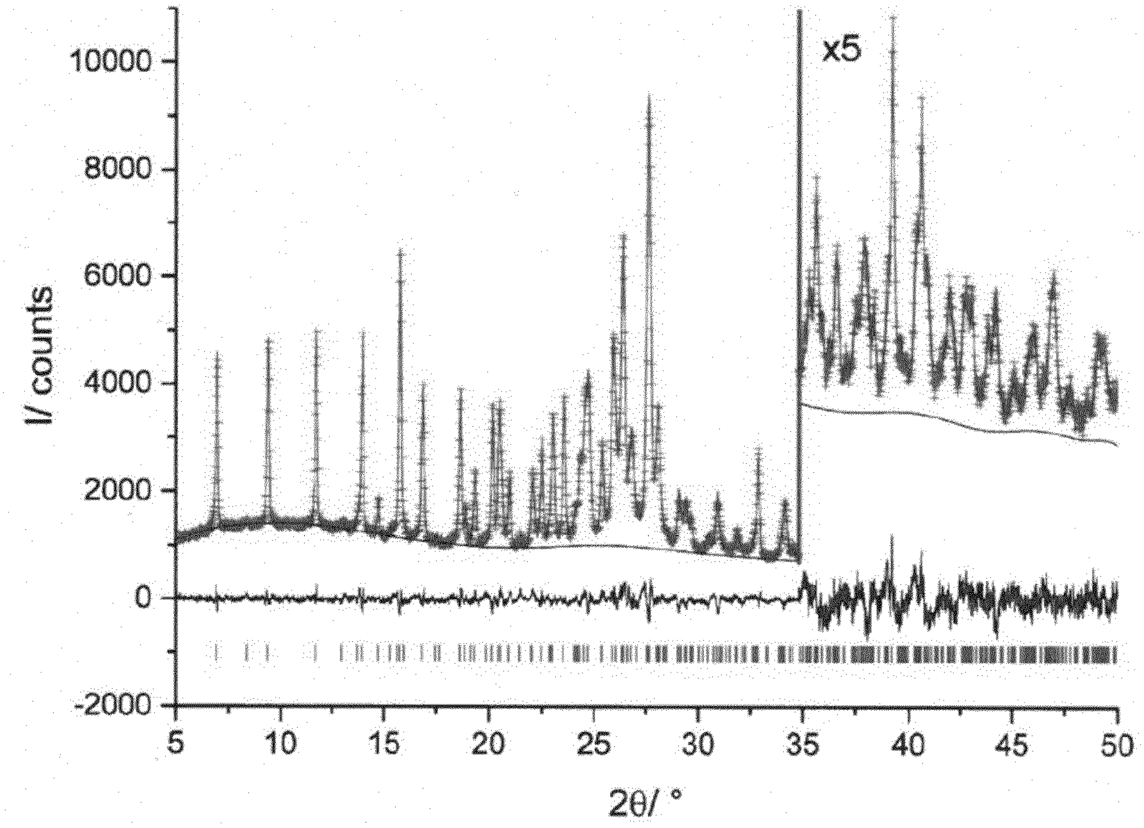

Figure 7

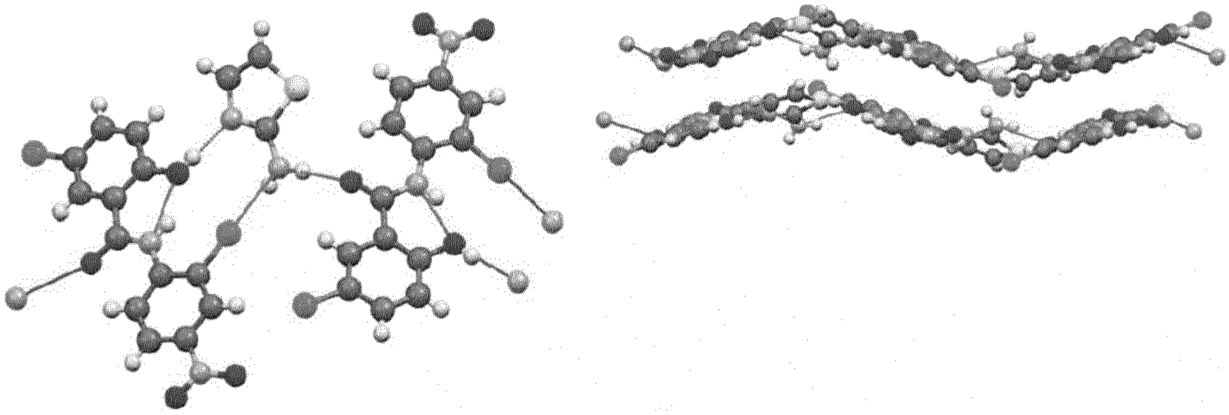

Figure 8

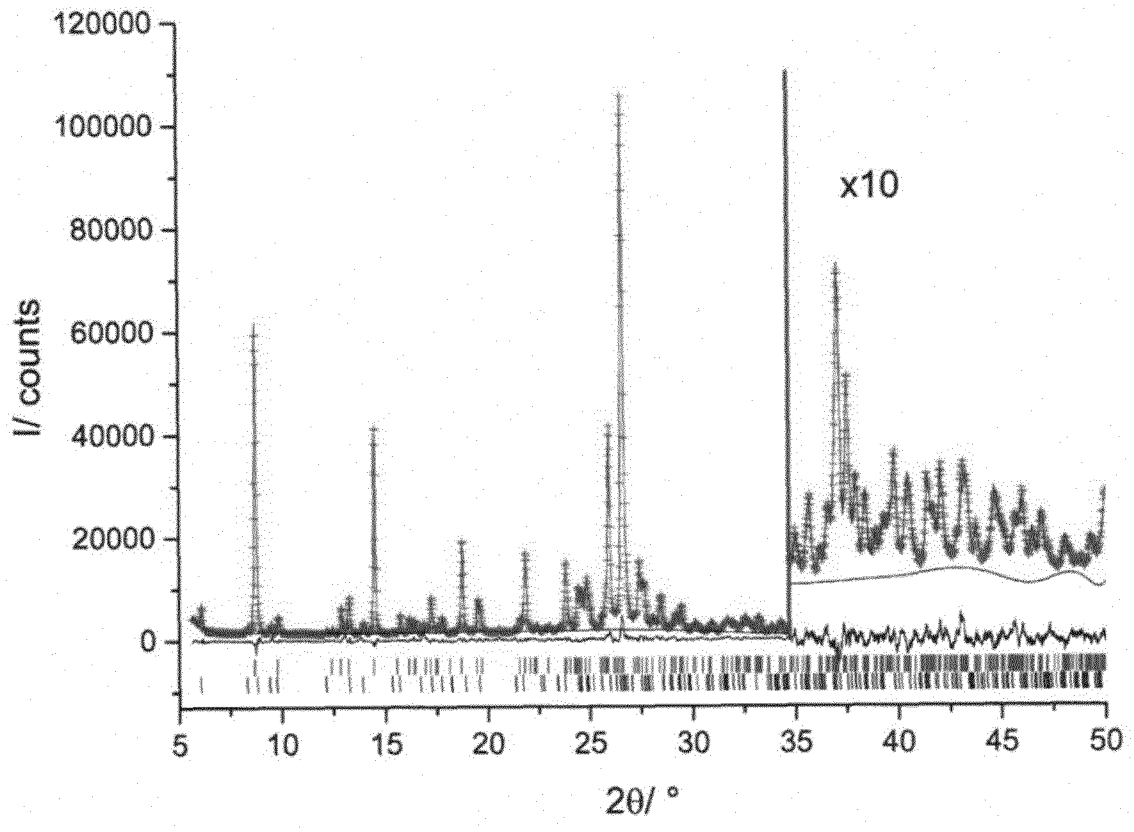

**Figure 9**

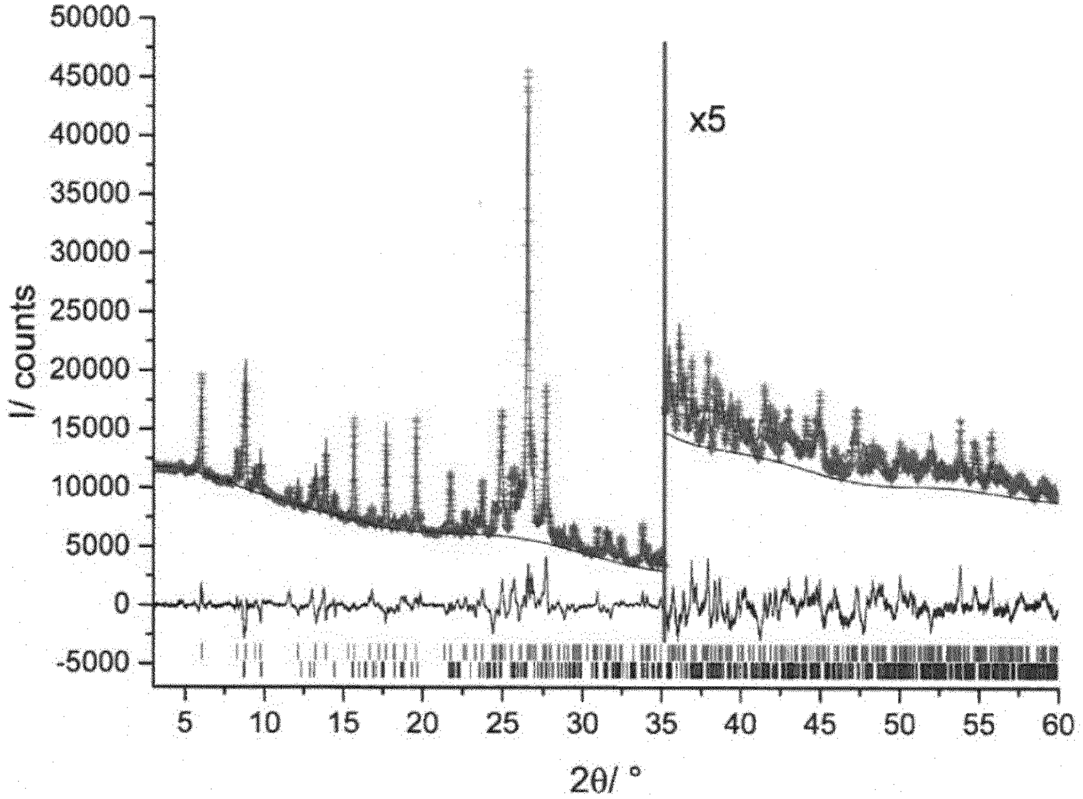


**Figure 10**

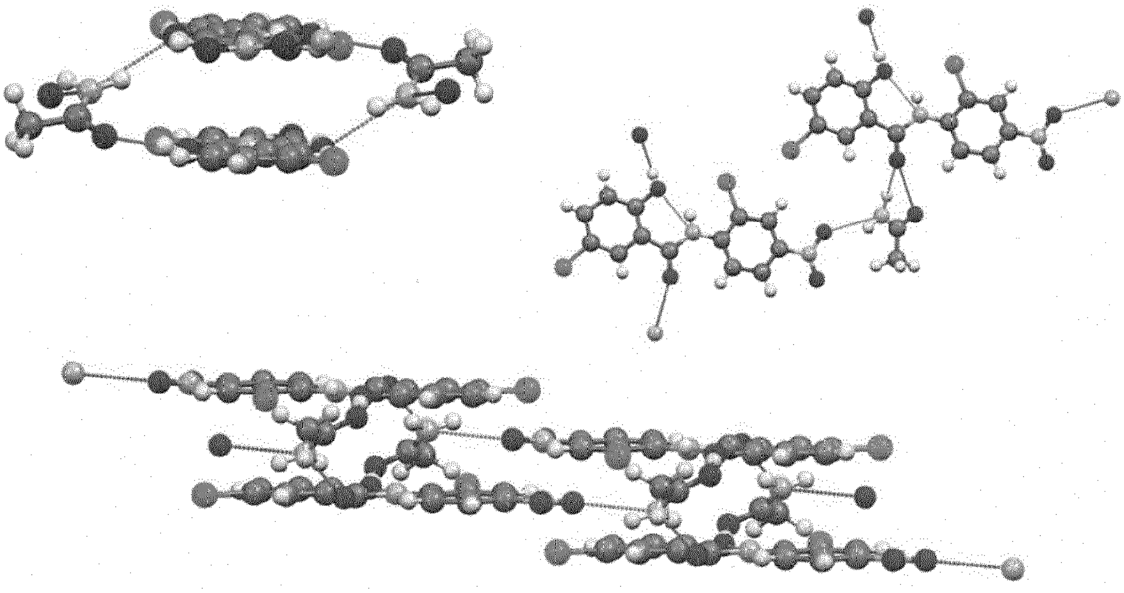

**Figure 11**

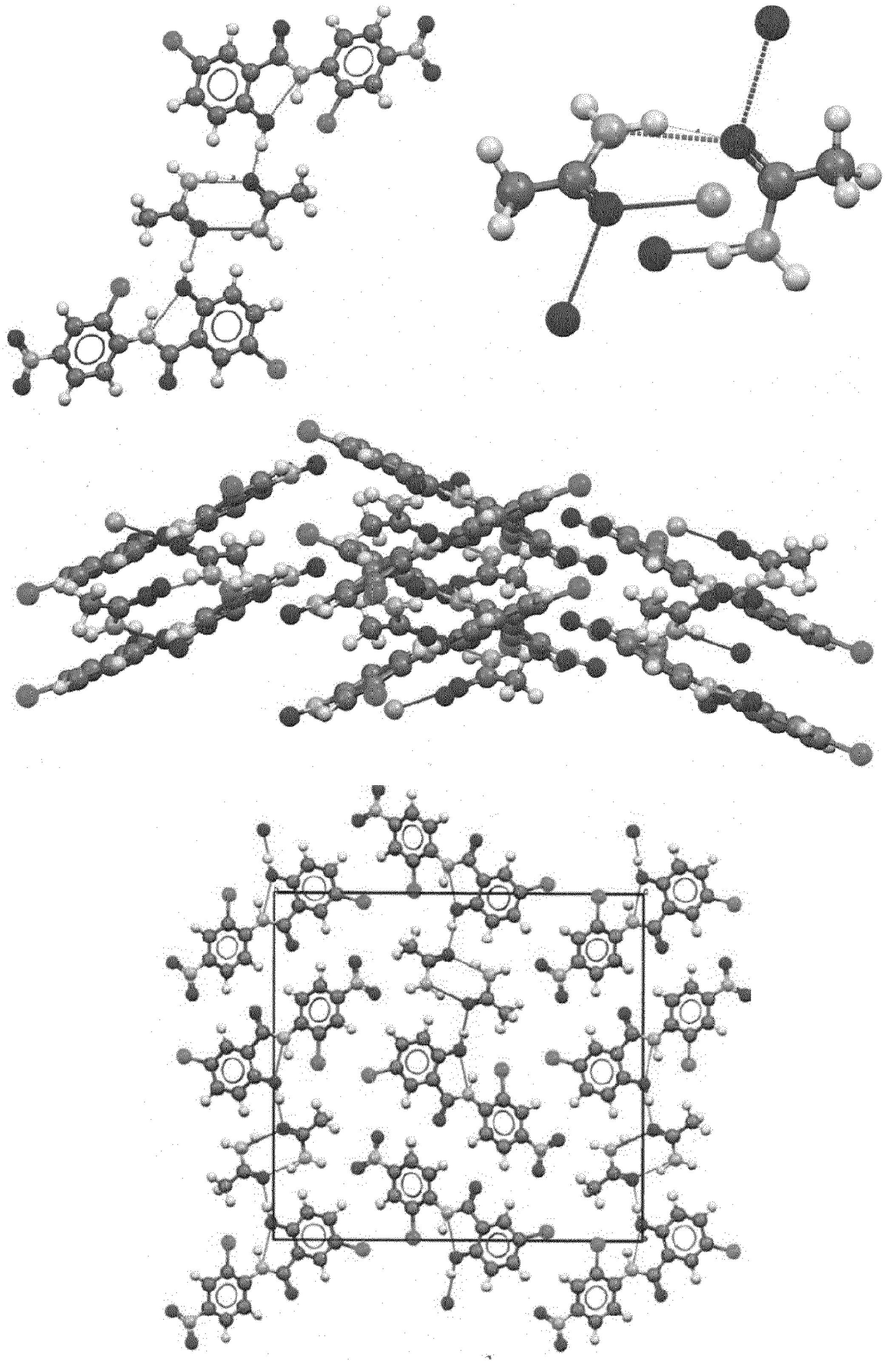

**Figure 12**

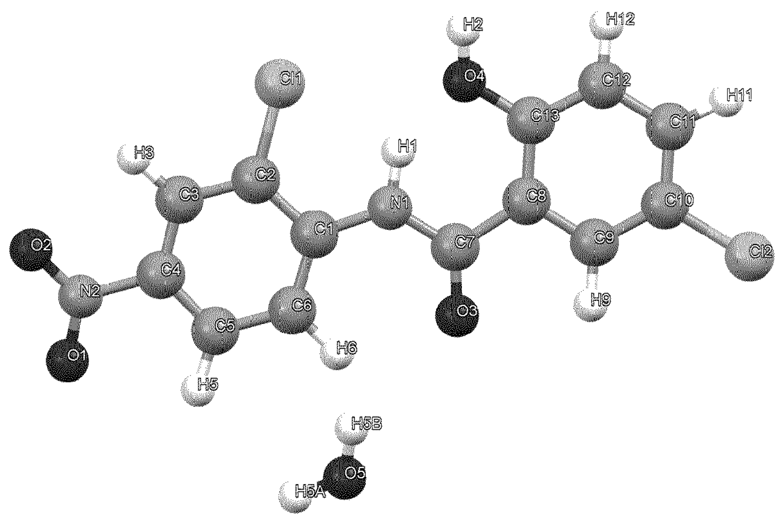

**Figure 13**

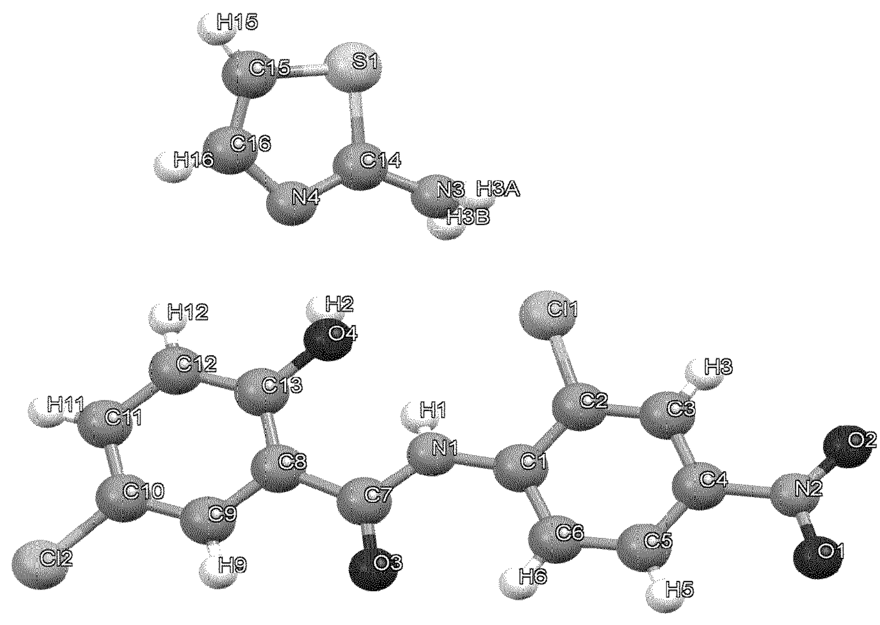

Figure 14

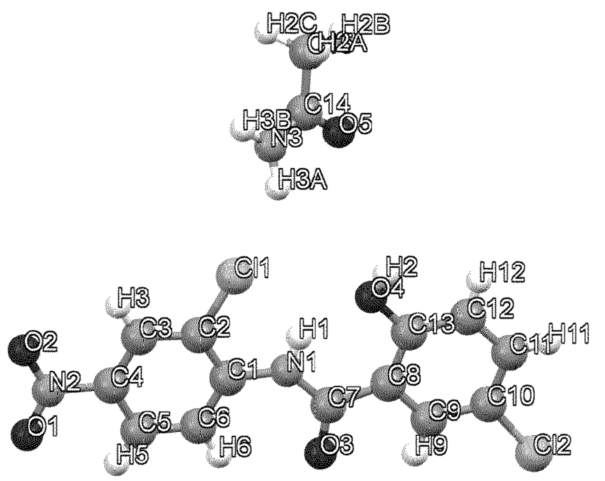

Figure 15

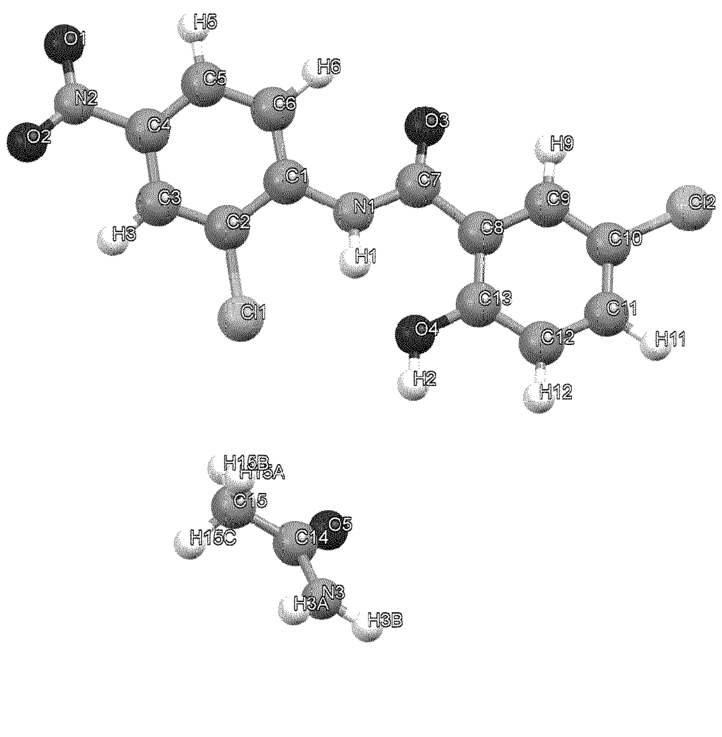

Europäisches Patentamt
European Patent Office
Office européen des brevets

# EUROPEAN SEARCH REPORT

Application Number
EP 15 19 3955

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | PALASH SANPHUI ET AL: "Pharmaceutical Cocrystals of Niclosamide", CRYSTAL GROWTH & DESIGN, vol. 12, no. 9, 5 September 2012 (2012-09-05), pages 4588-4599, XP055169367, ISSN: 1528-7483, DOI: 10.1021/cg300784v<br>* the whole document *<br>----- | 1-15 | INV.<br>C07D213/81<br>C07C235/64<br>C07D277/40<br>A61K31/167<br>A61P35/00<br>A61P25/28<br>A61P29/00 |
| X | FRANCESCA GRIFASI ET AL: "Using Salt Cocrystals to Improve the Solubility of Niclosamide", CRYSTAL GROWTH & DESIGN., vol. 15, no. 4, 1 April 2015 (2015-04-01), pages 1939-1948, XP055244077, US<br>ISSN: 1528-7483, DOI: 10.1021/acs.cgd.5b00106<br>* the whole document *<br>----- | 1-15 | |
| X,D | DE 10 2007 030695 A1 (SCICONCEPT GMBH [DE]) 8 January 2009 (2009-01-08)<br>* claims 1-3,16 *<br>----- | 1-15 | TECHNICAL FIELDS SEARCHED (IPC)<br>C07D<br>C07C |

The present search report has been drawn up for all claims

3

| Place of search | Date of completion of the search | Examiner |
|---|---|---|
| Munich | 26 January 2016 | Fanni, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 19 3955

This annex lists the patent family members relating to the patent documents cited in the above-mentioned European search report. The members are as contained in the European Patent Office EDP file on The European Patent Office is in no way liable for these particulars which are merely given for the purpose of information.

26-01-2016

| Patent document cited in search report | Publication date | Patent family member(s) | Publication date |
| --- | --- | --- | --- |
| DE 102007030695 A1 | 08-01-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- DE 102007030695
- WO 2005055983 A
- WO 2013040187 A

### Non-patent literature cited in the description

- **Y. LI et al.** *Cancer Letters,* 2014, vol. 349, 8-14
- **M.-Y. BAI et al.** *Colloids and Surfaces A: Physicochem. Eng. Aspects,* 2013, vol. 419, 248-256
- **P. SANPHUI et al.** *Crystal Growth & Design,* 2012, vol. 12, 4588-4599
- **F. GRIFASI et al.** *Crystal Growth & Design,* 2015, vol. 15, 1939-1948
- **D. LÜDEKER et al.** *Solid State Nucl. Magn. Reson.,* 2015, vol. 65, 29-40
- **D. LÜDEKER et al.** NMR crystallography. *Solid State Nucl. Magn. Reson.,* 2015, vol. 65, 29-40
- **P. SANPHUI et al.** *Crystal Growth & Design,* 04 December 2012, 588-4599